# EUROPEAN PATENT SPECIFICATION

(11) **EP 3 222 270 B1**
(45) Date of publication and mention of the grant of the patent: **29.04.2026**
(21) Application number: 16382136.6
(22) Date of filing: 23.03.2016
(51) Int. Cl.: A61K 9/00, A61K 9/06, A61K 47/10, A61K 47/38, A61K 47/02

(54) **COMPOSITIONS FOR MUCOSAL ADHESION AND USES THEREOF**
ZUSAMMENSETZUNGEN ZUR MUKOSALEN HAFTUNG UND VERWENDUNGEN DAVON
COMPOSITIONS POUR UNE ADHÉRENCE AUX MUQUEUSES ET LEURS UTILISATIONS

(43) Date of publication of application: 27.09.2017
(73) Proprietor: Bionanoplus, S.L., 31110 Noáin- Navarra (ES)
(72) Inventor: SALMAN, Hesham HA, E-31110 Noáin - Navarra (ES); PEÑALVA SOBRÓN, Rebeca, E-31110 Noáin - Navarra (ES); LLORENTE DOMÍNGUEZ, María, E-31110 Noáin - Navarra (ES); SÁNCHEZ GÓMEZ, Susana, E-31110 Noáin - Navarra (ES); GOÑI AZCÁRATE, Izaskun, E-31110 Noáin - Navarra (ES); LANZ AZPILICUETA, María, E-31110 Noáin - Navarra (ES); ESPARZA CATALÁN, Irene, E-31110 Noáin - Navarra (ES)
(74) Representative: ABG Intellectual Property Law, S.L.

(56) References cited:
- EP-A2- 0 306 454
- WO-A1-2005/027939
- WO-A1-2013/038402
- WO-A1-95/23591
- US-A1- 2014 249 118
- US-B1- 6 596 777

## Description

### TECHNICAL FIELD

The present invention relates to compositions which adhere to mucous membranes, particularly compositions of the type capable to hydrate and adhere immediately when come into contact with a mucous membrane.

### BACKGROUND ART

Adhesive compositions are sometimes used for the purpose of delivering active substances to a biological surface, for example as carrier systems for drug delivery applications. The ability of certain compositions to adhere to biological surfaces is known as bioadhesion, which may be defined as the state in which two materials, at least one of which is of biological nature, are held together for extended periods of time by interfacial forces. The biological surface can be epithelial tissue or a mucous membrane on the surface of a tissue. If the adhesive attachment is to a mucous membrane, the phenomenon is referred to as mucoadhesion. Mucous membranes include the mucosal linings of the oral, nasal, ocular, oesophageal, vaginal and rectal cavities.

Mucoadhesive substances are usually hydrophilic polymeric macromolecules which contain groups with capability of establishing hydrogen bonds. These substances are able to absorb water in contact with mucus, become hydrated and stick to the site of action. Mucoadhesion is a complex phenomenon comprising:
1. intimate contact between a bioadhesive and a membrane (hydration); and
2. penetration of the bioadhesive into the tissue or into the surface of the mucous membrane (interpenetration).

Hydration is required for a mucoadhesive polymer to expand and create a proper macromolecular mesh of sufficient size, and also to induce mobility in the polymer chains in order to enhance the interpenetration process between the mucoadhesive polymer and mucins, which are a family of high molecular weight, heavily glycosylated proteins present in the surface of the mucous membrane, forming a mucous network. Polymer hydrating permits a mechanical entanglement by exposing the bioadhesive sites for hydrogen bonding and/or electrostatic interaction between the polymer and the mucous network. Mucoadhesive polymers with these characteristics are also called swelling agents. Importantly, a critical degree of hydration, swelling or solvation of the swelling agent exists where optimum hydration and adhesion occurs. In fact, over-hydration of the polymer results in the formation of slippery mucilage with no adhesion. For this reason, gels or pastes comprising hydrophilic mucoadhesive polymers hydrated in water or solvated in polyols have poor mucoadhesive properties in comparison with tablets where the polymers are in dry form and only hydrate when in contact with biological fluids, thereby resulting in strong adhesion to the mucous layer.

The presence of water as an ingredient of adhesive compositions limits the utility of these products. Therefore, adhesive compositions that do not require the addition of water to be manufactured are preferably desired.

Currently, the drug delivery adhesive vehicle of reference for oral mucous membranes is an oral paste sold under the name Orabase^{®}. This product is composed of gelatin, pectin and sodium carboxymethylcellulose in an oily vehicle containing polyethylene glycol and paraffin known as plastibase^{®}. Orabase^{®} is presented as an odorless, bitter, hydrophobic, oily mass. The main advantage of this product is that its mucoadhesive polymers are neither pre-hydrated nor swelled or solvated, and therefore they are able to become hydrated in the site of administration, thus maintaining all their adhesive potential. However, despite being the adhesive paste of reference, its high oil content (45-50 %) generates slow water (or salivary fluid) penetration necessary for the hydration of the polymers. This results in an oily, earthy and clayey mouthfeel. In addition, oils are immiscible with the mucin layer and saliva, which reduces its residence time and generates unpleasant lumps in the mouth.

Patent US 4,894,232 describes an anhydrous base for mucosal and denture adhesive pastes comprising a solid hydratable agent which is hydrated only in water. It is dispersed in a colloidal solution of an organic second swelling agent in a water-soluble, physiologically tolerated organic solvent, wherein the organic second swelling agent is hydrated in water and is an ionic swelling agent such as xanthan gum. In this situation, the adhesive polymer remains non-hydrated and dispersed in the medium, therefore maintaining its adhesive properties. However, if the adhesive polymer is merely dispersed in the medium it will eventually precipitate, which compromises the long term stability of the product. The only possibility to stabilize this composition is to increase the viscosity of the organic solution with the second swelling agent. For this reason, this product is only useful for high viscosity formulations, and not for low viscosity solutions (< 40 Pa.s) more adequate for a general dispersion in the mouth or other administration routes such as vaginal. Moreover, this product would only be used with adhesive polymers that are only swellable in water and not in the organic solvent (i.e. glycerol). Thus, the use of this product is limited to a few polymers (i.e. tragacanth gum, locust bean gum, galactomannans and guar gum) excluding hydrophilic polymers with the highest mucoadhesive properties such as sodium carboxymethyl cellulose (CMC), because this polymer swells in polyol solvents, therefore losing its mucoadhesive properties.

Patent application US 2015/0017105 A1 has recently described alternative anhydrous hydrophilic gel formulations as mucoadhesive drug delivery vehicles. These gel formulations are obtained by combining a biocompatible polymer (CMC, pectin, alginate, carrageenan, gums, polyaspartic acid, polyglutamic acid, hyaluronic acid, etc.) with a polyol (ethylene glycol, propylene glycol, glycerol, sorbitol, mannitol, methyl glucoside, etc) and introducing the resulting mixture in an oven (under suitable dry conditions so as to retain a water free atmosphere) set at 105 °C for about 5 minutes until the mixture sets. The adhesive properties of this gel are limited, so the hydrophilic polymers are swelled in the polyol.

US 6 596 777 B1 discloses compositions for application to a mammalian biomembrane comprising a polyhydroxy compound ranging from about 0.5 to about 25%, a crosslinked polymer, a water soluble non-crosslinked polymer and about 30% to about 98% water.

EP 0 306 454 A2 discloses anhydrous tablets having improved bioadhesion to mucous membranes comprising a water-soluble natural biopolymer such as xanthan gum, pectin or mixtures thereof; and a solid polyol. Some of the tablets disclosed contain calcium stearate. It is also disclosed that the tablets can optionally contain sodium or calcium saccharin salts.

US 2014/249118 A1 discloses a bioadhesive paste comprising triamcinolone acetonide and retinoic acid in the commercial composition Orabase^{®} that contains gelatin, pectin, carboxy methylcellulose sodium and plasticized hydrocarbon gel such as polyethylene and mineral oil gel base. Said composition is used for reducing or eliminating intraoral inflammation of oral lichen planus when topically administered on an affected area of the mucosal lesions.

Therefore, there is a need in the art to obtain a highly adhesive hydrophilic anhydrous composition, capable to hydrate and adhere immediately when administered to a mucous membrane. This composition should have high residence time, improved taste and long term stability.

### SUMMARY OF THE INVENTION

The present invention refers to a mucoadhesive composition with improved characteristics which make it suitable for use as adhesive vehicle for mucous membranes, for example as a drug delivery agent for mucous membranes. Particularly, the mucoadhesive composition of the invention has long term stability, high capacity of maintaining their bioadhesive properties over time, high hydration at the contact site, high residence time and pleasant mouthfeel.

It is generally known that the adhesive properties of a composition containing a swelling polymer diminish proportionally to its viscosity increase, which is due to the polymer swelling in the composition.

The inventors have surprisingly found that the dispersion of a divalent salt, particularly an inorganic salt comprising divalent cations selected from the group consisting of CaSO₄, CaCl₂, MgCl₂, CuSO₄, CaCO₃, or a mixture thereof, in a composition comprising at least a polyol selected from the group consisting of glycerol, propylene glycol and a combination thereof, before the addition of at least a swelling polymer such as cellulose or a derivative thereof as defined in claim 1 to said composition, avoids the swelling of the polymer in the polyol but not the swelling of the polymer in an aqueous media, thus avoiding the increase of the viscosity in the mixture and, then, maintaining the bioadhesive properties of the composition over time.

Particularly, the inventors have surprisingly found that the optimum ratio (w/w) swelling polymer/divalent salt in a composition comprising at least a polyol, at least a swelling polymer selected from celluloses or a derivative thereof and at least an inorganic salt comprising divalent cations is from 4 to 250. Compositions with this ratio maintain their adhesive properties over time because the swelling polymer is dispersed in the solvent and not swelled.

Additionally, the inventors have observed that some of said compositions show a sedimentation effect of the cellulose derivative, thus obtaining a composition with phase separation. The inventors of the present invention have surprisingly found that when a solid waxy surfactant selected from the group consisting of glyceryl monostearate, sorbitan monostearate, cetostearyl alcohol and combinations thereof is dispersed or emulsified in the composition before the addition of the swelling polymer, the sedimentation effect of the cellulose derivative is significantly decreased, thus decreasing phase separation. Particularly, said decrease is proportional to the amount of solid waxy surfactant added. More particularly, for compositions with less than 15% w/w of CMC and solid waxy surfactant concentration from 1 to 3% w/w, no phase separation is observed.

The compositions of the invention show better adhesive properties than the commercial adhesive product Orabase^{®}, which also contains a derivative of cellulose and a polyol, and are capable of maintaining said adhesive properties over time.

Furthermore, the inventors have observed that the addition of a solid waxy surfactant selected from the group consisting of glylceryl monostearate, sorbitan monostearate, cetostearyl alcohol and combinations thereof increases the residence time of the adhesive compositions in their target site of adhesion. This is because the solid waxy surfactant decreases the hydrophilic properties of the composition to the necessary proportion to guarantee the entry of a quantity of saliva suitable for hydrating and adhering the composition to the mucous membrane without allowing the dissolution of the composition or their partial loss from the site of adhesion (see Example 3).

Therefore, the composition of the present invention comprises, as essential elements, a polyol or a mixture thereof, a swelling polymer selected from the group consisting of celluloses and a derivative thereof or a mixture thereof, an inorganic salt comprising divalent cations or a mixture thereof, and a solid waxy surfactant or a mixture thereof as defined in claim 1.

In a first aspect, the invention relates to a composition having a dynamic viscosity from 4 to 300 Pa.s comprising:
(i) a polyol selected from the group consisting of glycerol, propylene glycol and a combination thereof in an amount of at least 60 wt %;
(ii) a swelling polymer selected from the group consisting of celluloses and a derivative thereof or a mixture thereof in an amount of at least 1 wt %, wherein the derivative of celluloses is selected from the group consisting of a salt of carboxymethyl cellulose (CMC), ethylcellulose, hydroxyethyl cellulose, hydroxyethylmethyl cellulose, hydroxypropyl methylcellulose (HPMC) or hypromellose or a derivative thereof, hydroxypropyl cellulose (HPC), low-substituted hydroxypropyl cellulose (L-HPC), methylcellulose, and a combination thereof;
(iii) an inorganic salt comprising divalent cations selected from the group consisting of CaSO₄, CaCl₂, MgCl₂, CuSO₄, and CaCO₃, or a mixture thereof in an amount ranging from 0.01 to 5 wt %; and
(iv) a solid waxy surfactant selected from the group consisting of glylceryl monostearate, sorbitan monostearate, cetostearyl alcohol and combinations thereof
wherein said composition does not comprise more than 3.5 wt % of water, wherein wt % is the weight of each component relative to the total weight of the composition and wherein the ratio component (ii)/component (iii) is from 4 to 250; and wherein the dynamic viscosity is measured at a temperature of 19 ± 2 °C and at 12 rpm as rotational speed.

The content in water of the composition of the invention is not due to the addition of water as an ingredient, but to low quantities of water present in components (i), (ii), (iii) and/or (iv) of the composition, if any.

It is generally known that most hydrophilic swelling polymers become hydrated in water or solvated/swelled in organic solvents such as polyols. Until now, the only possibility to use a hydrophilic swelling polymer in unhydrated/unsolvated/unswelled form, dispersed in an anhydrous medium (adequate for oral, buccal, vaginal or rectal administration) was to either use a swelling polymer that is only solvatable in water (therefore limiting the available swelling agents to a reduced number of polymers with limited bioadhesive capacity), or use an oily medium where the polymer will not become hydrated/ solvated.

It is also well known that hydrophilic polymers can interact with polyvalent cations and this interaction can affect their swelling and adhesive capacity, viscosity and solubility. For example, carrageenan or alginate can interact with a calcium salt in an aqueous medium and form a cross linked hydrogel. However, this effect is not observed when the medium is a polyol instead of water, since neither the polysaccharide nor the calcium salt are soluble or solvatable enough in this medium. In the case of other polymers such as cellulose derivatives or xanthan gum, which become hydrated in water and form hydrogels, it is documented that polyvalent cations do not form cross linked gels with such polymers. Thus, CMC and xanthan gum are capable of forming hydrogels independently of the presence of divalent cations. However, it has been reported that viscosity variations can occur when polyvalent cations are added to a CMC or xanthan gum water solution, and these variations may be accompanied by haze formation.

In view of all this, it was to be expected that swelling polymers such as CMC or xanthan gum would not interact with a divalent cation such as calcium in anhydrous polyol solvents due to the limited solubility of calcium salts in such solvents and that both polymers would form a gel.

Surprisingly, the inventors have found that dispersing an inorganic salt comprising divalent cations selected from the group consisting of CaSO₄, CaCl₂, MgCl₂, CuSO₄, CaCO₃, or a mixture thereof, in an anhydrous polyol solvent selected from the group consisting of glycerol, propylene glycol and a combination thereof, before adding CMC, prevented the swelling polymer from becoming solvated in the polyol (thus a suspension of CMC in the polyol solvent was obtained, and not a gel), without affecting the adhesive properties of the swelling polymer. Further, the inventors found that suspensions of CMC in a polyol selected from the group consisting of glycerol, propylene glycol and a combination thereof with different amounts of a divalent salt selected from the group consisting of CaSO₄, CaCl₂, MgCl₂, CuSO₄, CaCO₃, or a mixture thereof, were stable for at least one month at both 25 °C and 40 °C while the same compositions without the salt formed gels that increased their viscosity after one day of storage, reaching a solid mixture in a few days (depending on the initial amount of CMC). Additionally, they confirmed that, in the presence of salts, the polymer became hydrated and its adhesive properties remained unaltered when in contact with salivary fluids or water, despite the presence of divalent salts. This is due to the fact that, when the composition contains divalent salts, the swelling polymer is not solvated but dispersed, therefore keeping its adhesive properties. In contrast, when no divalent salts are present in the composition, the adhesive properties of the mixture decrease as the viscosity increases due to the swelling of the swelling polymer as it becomes solvated.

Moreover, the inventors surprisingly found that if a hydrophilic swelling polymer such as CMC was added to a polyol selected from the group consisting of glycerol, propylene glycol and a combination thereof comprising a divalent salt selected from the group consisting of CaSO₄, CaCl₂, MgCl₂, CuSO₄, CaCO₃, or a mixture thereof, and additionally comprising a waxy surfactant selected from the group consisting of glyceryl monostearate, sorbitan monostearate, cetostearyl alcohol and combinations thereof, a significant longer residence time to the mucosa was observed in comparison with formulations without such waxy surfactant or without such waxy surfactant and the calcium salt.

Further, the use of such waxy surfactant avoided the formation of two phases which sometimes appear due to the sedimentation of the unhydrated/ unsolvated swelling polymer. However, in those cases where slight sedimentation or separation was observed, the addition of thickening agents such as xanthan gum increased the viscosity and avoided the swelling polymer precipitation without any significant modifications of the viscosity over time due to the surprising effect of divalent salts. This allows for the preparation of highly stable formulations.

Hence, it was possible to obtain an unhydrated/ unsolvated/ unswelled hydrophilic swelling polymer dispersed in an anhydrous organic medium. The inventors also surprisingly confirmed that such swelling polymer remains hydratable in water and in biological fluids, so the composition maintains high mucoadhesive properties with a surprisingly stable and long lasting effect, particularly if a solid waxy surfactant selected from the group consisting of glyceryl monostearate, sorbitan monostearate, cetostearyl alcohol and combinations thereof, is added to the formulation. In fact, it was confirmed that the mucoadhesive capacity of this composition is significantly higher than that of conventional pastes and hydrogels, including Orabase^{®} formulations also containing CMC. This improved adhesive potential is due to the fact that the composition of the invention is hydrophilic, so water can rapidly penetrate into the mixture and hydrate the unhydrated swelling polymers (which therefore keep all their adhesive potential), avoiding the formation of not adherent lumps. Additionally, if solid waxy surfactants have been added to the composition, their presence is capable of limiting the amount of water that can rapidly penetrate into the mixture to just the amount of water necessary to hydrate the adhesive polymer without dissolving the product, therefore achieving maximum residence times.

Moreover, it is possible to incorporate into the base composition (comprising the polyol, the inorganic salt comprising divalent cations, the hydrophilic swelling polymer and the solid waxy surfactant as defined in claim 1) different thickeners, polysaccharides different from celluloses or derivatives, adhesive polymers, fats and surfactants different from the solid waxy surfactant of the composition, therefore controlling the viscosity and the physicochemical properties of the final product. It is also possible to incorporate into such base active substances that can be used topically.

The resulting composition is an anhydrous formulation with improved cosmetic and organoleptic properties compared with the Orabase^{®} formulation of reference, where the composition has a smooth and creamy texture and no lumps are formed once in contact with mucosa.

The mucoadhesive composition described above may be used as a medicament, particularly as a vehicle for drug delivery to mucous membranes. It may be useful in the prevention and/or treatment of a buccal, gastric/ gastroesophageal, anal or vaginal disease.

In a second aspect, the present invention relates to the composition of the invention further comprising at least an active ingredient for use as a medicament.

In a third aspect, the invention relates to the composition of the invention further comprising at least an active ingredient for use in the prevention and/or treatment of a disease selected from the group consisting of:
(a) a buccal disease selected from the group consisting of xerostomia, keratosis, desquamative stomatitis (or gingivitis), candidiasis, pericoronitis, erythroplasia, burning mouth syndrome, cancer sores, cold sores, geographic tongue, leukoplakia, lichen planus, erythema multiforme, drug eruptions, halitosis, periodontitis, pemphigus vulgaris, Sjögren's syndrome, gingivitis, ulceration, recurrent aphtous stomatitis and malignant ulcers;
(b) a gastric/gastroesophageal disease selected from the group consisting of gastroesophageal reflux and esophagitis;
(c) an anal disease that is the haemorrhoidal disease; and
(d) a vaginal disease selected from the group consisting of bacterial vaginosis, vaginal yeast infection, vaginitis, chlamydia, gonorrhea, genital herpes, and genital warts.

The mucoadhesive composition may also be used for non medical purposes, such as an adhesive for dentures.

In a fourth aspect, the invention relates to the use of the composition according to the invention as an adhesive for dentures.

In another aspect, the invention provides a method for preparing the mucoadhesive composition of the invention comprising:
(a) dispersing component (iii) and component (iv) within component (i); and
(b) dispersing component (ii) in the mixture resulting from the previous step.

In another aspect, the invention relates to a method for obtaining a nanoparticle composition comprising contacting a composition according to the invention that contains an adhesive polymer dissolved in compound (i), with an aqueous medium, wherein said adhesive polymer is selected from the group consisting of half esters of poly(methyl vinyl ether-co-maleic anhydride) (PVM/MA) copolymers, poly(methylvinyl ether/maleic acid), zein and polymethacrylates.

### BRIEF DESCRIPTION OF THE DRAWINGS

**Fig. 1****.** Adhesion time of the different formulations tested. F696, formulation with divalent salt and waxy surfactant; F621, formulation with divalent salt and without waxy surfactant; F575, formulation with waxy surfactant and without divalent salt; F576, formulation without divalent salt and waxy surfactant. min, minutes
**Fig. 2****.** Miconazole levels in human saliva over time. Comparison of mouth residence time of miconazole for two different formulations: Daktarin^{®} and F696. h, hours.
**Fig. 3****.** *In vitro* release-adhesion of carbenoxolone for two different formulations: Sanodin^{®} and F696. h, hours.
**Fig. 4****.** *Ex vivo* adhesion in horse oesophagi of two different formulations: F2-E and Daktarin^{®}. A) Comparation between Daktarin^{®} and F2-E at different time points. B) Formulation adhesion in oesophagus cut at different time points. min, minutes.
**Fig. 5****.** Evolution of the adhesion properties of a formulation according to the invention depending on the formulation viscosity. min, minutes.

### DETAILED DESCRIPTION

The present invention provides a mucoadhesive composition as defined in claim 1. The present invention also provides methods for obtaining the mucoadhesive composition of the invention and describes uses thereof.

### Definitions

In order to facilitate the comprehension of the present invention, the meaning of some terms and expressions as used in the context of the invention are set forth below.

As used herein, the term "viscosity" relates to the resistance of a fluid to flow. Viscosity is a measure of the resistance of a fluid to gradual deformation by shear stress or tensile stress. Fluids with low viscosity are typically in the gaseous or liquid state. Fluids with high viscosity may appear to be solid. In the context of the present invention, the viscosity of the compositions is the dynamic viscosity and was measured with a digital viscometer (Viscotech Hispania, V1-R) by controlling different critical parameters: sample temperature, spindle characteristics and rpm. The viscosity was measured at a temperature of 19 ± 2 °C, with a specific spindle (Spindle PD or viscosities between 10 Pa.s and 170 Pa.s and Spindle PF for viscosities between 50 Pa.s and 830 Pa.s), and at 12 rpm as rotational speed.

As used herein, the term "polyol" relates to an alcohol comprising multiple hydroxyl groups (at least two hydroxyl groups). Liquid polyols are used as water free solvents in the context of the present invention to prevent a swelling polymer from becoming hydrated. The polyol used in the compositions of the invention can be anhydrous or with less than 2 wt% water content, preferably with less than 1 wt% water content.

As used herein, the term "aqueous medium" relates to a solution in which the solvent is water. Said aqueous medium may comprise water and a water miscible solvent. Substances that are hydrophobic ('water-fearing') often do not dissolve well in water, whereas those that are hydrophilic ('water-loving') do.

As used herein, the term "cellulose" relates to a polysaccharide consisting of a linear chain of several hundred to many thousands of β(1→4) linked D-glucose units.

As used herein, the term "cellulose derivative" relates to compounds resulting from the chemical reaction between the available hydroxyl groups of cellulose and another molecule. Typical cellulose derivatives are the esters and ethers of cellulose.

As used herein, the term "inorganic salt" relates to an ionic compound composed of correlated numbers of positively charged ions (cations) and negatively charged ions (anions) so that the resulting product is electrically neutral, where the positively charged ions do not contain carbon atoms and where the negative charged ions do not include neither hydroxide (OH⁻) nor oxide (O²⁻) ions. Preferably, the inorganic salt of the invention has a pKa lower than 11.

As used herein, the term "divalent cations" relates to a positively charged ion which has a charge of +2.

As used herein, the term "emulsified" or "emulsion" relates to a mixture of two immiscible liquids wherein a first liquid is dispersed within a second liquid, the two liquids being incapable of forming a homogenous phase.

As used herein, the term "mucoadhesive" relates to a substance capable of adhering to mucous membranes. In the context of the present invention, the mucoadhesive composition is capable of adhering to a mucous membrane immediately after entering into contact with said mucous membrane due to the *in situ* hydration of the swelling polymer by the biological fluid (mucus) present in said membrane. However, the composition of the invention as such is not adhesive before contacting the mucous membrane. Mucous membranes include, without limitation, the mucosal linings of the oral, nasal, ocular, oesophageal, vaginal and rectal cavities. The compositions of the invention can adhere to any mucous membrane. Preferably, the compositions of the invention adhere to the mucous membrane of the oral, oesophageal, vaginal or rectal cavities. The mucoadhesive properties of a composition depend on the viscosity. The compositions of the invention having a viscosity from 4 to 300 Pa.s show an optimal adhesion.

As used herein, the terms "topic" or "topical" relate to substances to be applied on specific surfaces of the body, as opposed to substances which are applied systemically throughout the body. Typically, topical application includes application on the skin or mucous membranes. The compositions of the invention are applied topically. In the context of the present invention the topical application relates to application on mucous membranes.

As used herein, the term "buccal" relates to substances to be applied on or inside the mouth, or related to this part of the body.

As used herein, the term "fat" relates to esters of three fatty acid chains and the alcohol glycerol. Fat is a general term and it also comprises oils. Oils are fats wherein the fatty acid chains are short or unsaturated, and which are liquid at room temperature.

As used herein, the term "tensioactive", or "surfactant", relates to compounds capable of lowering the surface tension between two liquids. Tensioactive compounds may be used as dispersants or emulsifiers.

As used herein, the term "medicament" relates to an agent in a specified formulation that promotes recovery from injury or ailment; or prevents disease.

As used herein, the terms "prevent," "preventing," and "prevention", refer to inhibiting the inception or decreasing the occurrence of a disease in a subject. Prevention may be complete (e.g. the total absence of pathological signs in a subject) or partial. Prevention also refers to a reduced susceptibility to a clinical condition.

As used herein, the terms "treat" or "treatment" relate to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

As used herein, the term "anhydrous" relates to a compound or composition without water, particularly water of hydration, or having a low quantity of water that comes from the raw materials used (from components (i), (ii), (iii) and/or (iv)) but is not added as a specific component of the composition. For example component (i) (polyol) may have 2 wt% water, thus making that the final composition of the invention has a low quantity of water. Taking into account the possible content of water of components (i), (ii), (iii) and (iv), the maximum quantity of water than can have the composition of the invention is 3.5 wt%. In the context of the present invention, the anhydrous composition has a water content below 3.5 wt%, preferably below 3.0 wt%, more preferably below 2.5 wt%, more preferably below 2.0 wt%, even more preferably below 1.5 wt%, even more preferably below 1 wt%, even more preferably below 0.5 wt% and still more preferably the water content is negligible or 0%.

As used herein, the term "hydrated" relates to a compound which is chemically combined with water, especially existing in the form of a hydrate.

As used herein, the term "solvated" relates to a compound which is chemically combined with a solvent, especially existing in the form of a solvate.

As used herein, the term "polymer swelling" relates to the increase of volume of a polymer due to absorption of a solvent (either water or another solvent).

As used herein, the term "polymer hydration" relates to the hydrogel structure created by the hydrophilic groups or domains present in a polymeric network upon the introduction of the polymer in an aqueous environment or upon contacting the polymer with an aqueous environment. After contacting with an aqueous environment, the polymer has to swell to become adhesive and adhere to the contact site.

As used herein, the term "swelling polymer" relates to a three-dimensional network of hydrophilic polymer chains that are chemically or physically cross-linked. Depending on their structure, swelling polymers can absorb aqueous or organic solutions. If the former is the case, a swelling polymer is called a hydrogel. Since most swelling polymers are of hydrophilic nature, they can absorb significant amounts of gaseous water (moisture) or liquid water. Hydrogels may increase from 10 to 1,000 times their own weight when placed in an aqueous medium.

As used herein, the term "solid waxy surfactant" relates to a tensioactive compound, which is presented as creamy unctuous masses, flakes, pellets, granules or powder at a temperature of 17-25 °C. Each solid waxy surfactant has a different melting temperature that usually ranges from 30 to 70 °C. Exemplary solid waxy surfactants are, without limitation, glyceryl monostearate, glyceryl behenate, lauroyl polyoxylglycerides, stearoyl polyoxylglycerides, sorbitan monostearate, sorbitan monopalmitate, cetostearyl alcohol, lauric acid, macrogol-15-hydroxystearate, myristyl alcohol, polyoxyethylene alkyl ethers (Polyoxyl 6 cetostearyl ether, Polyoxyl 23 lauryl ether, Polyoxyl 2 cetyl ether, Polyoxyl 10 cetyl ether, Polyoxyl 20 cetyl ether, Polyoxyl 10 stearyl ether), polyoxyethylene stearates (Polyoxyl 8 stearate, Polyoxyl 20 stearate, Polyoxyl 40 stearate), or emulsifying wax (non ionic).

As used herein, the term "thickener" relates to a substance which can increase the viscosity of a liquid without substantially changing its other properties. Thickeners may also improve the suspension of other ingredients or emulsions which increases the stability of the product. Exemplary thickeners are, without limitation, copovidone, carbomer, polyethylene oxide, polyethylene glycol, a polysaccharide different from celluloses or a derivative thereof (such as sodium alginate, sodium carragenate, pectine, guar gum, xanthan gum, acacia gum, gellan gum, tragacanth gum, agar-agar, starch) and silica.

As used herein, the term "polysachharide" relates to a polymeric carbohydrate molecule composed of long chains of monosaccharide units bound together by glycoside linkages that give the constituent monosaccharides or oligosaccharides by hydrolysis. They range in structure from linear to highly branched.

As used herein, the term "adhesive polymer" relates to a large molecule or macromolecule composed of many repeated subunits capable of adhering to a surface and that is capable of forming *in situ* nanoparticles after being dissolved in a polyol when in contact with an aqueous medium. Exemplary adhesive polymers are, without limitation, half esters of poly(methyl vinyl ether-co-maleic anhydride) (PVM/MA) copolymers, poly(methylvinyl ether/maleic acid), zein and polymethacrylates.

As used herein, the term "unstable formulation" relates to compositions:
a) whose viscosity changes considerably after 4 days at 40°C, preferably after one week at 40 °C, preferably after 2 weeks at 40°C, preferably after 4 weeks at 40°C, more preferably after 6 weeks at 40°C, even more preferably after 1 hour at 70 °C or
b) showing phase separation.

In the context of the present invention if the composition has an initial viscosity ≤ 70 Pa.s, said composition is considered unstable when the viscosity after 4 days at 40°C, preferably after one week at 40°C, preferably after 2 weeks at 40°C, preferably after 4 weeks at 40°C, more preferably after 6 weeks at 40°C, even more preferably at 1 hour at 70°C is higher than 110 Pa.s. In the context of the present invention if the composition has an initial viscosity >70 Pa.s, said composition is considered unstable when its viscosity deviation from the initial viscosity measurement after 4 days at 40°C, preferably after one week at 40°C, preferably after 2 weeks at 40°C, preferably after 4 weeks at 40°C, more preferably after 6 weeks at 40°C, even more preferably at 1 hour at 70°C is higher than ± 40 Pa.s. Furthermore, in the context of the present invention, a formulation is considered unstable when it shows phase separation into two different phases to the naked eye (a solid phase due to cellulose sedimentation and a liquid phase containing polyol, being the liquid phase higher than 5 % wt of such polyol) and said phase separation, despite being reduced or eliminated when shaking, reappears over time.

As used herein, the term "stable formulation" relates to compositions:
a) whose viscosity does not change considerably after 4 days at 40°C, preferably after one week at 40 °C, preferably after 2 weeks at 40°C, preferably after 4 weeks at 40°C, more preferably after 6 weeks at 40°C, even more preferably after 1 hour at 70 °C and
b) whose phase separation is negligible.

In the context of the present invention if the composition has an initial viscosity ≤ 70 Pa.s, said composition is considered stable when viscosity after 4 days at 40°C, preferably after one week at 40°C, preferably after 2 weeks at 40°C, preferably after 4 weeks at 40°C, more preferably after 6 weeks at 40°C, even more preferably at 1 hour at 70°C remains between 4 and 110 Pa.s. In the context of the present invention if the composition has an initial viscosity >70 Pa.s, said composition is considered stable when its maximum viscosity deviation from the initial viscosity measurement after 4 days at 40°C, preferably after one week at 40°C, preferably after 2 weeks at 40°C, preferably after 4 weeks at 40°C, more preferably after 6 weeks at 40°C, even more preferably at 1 hour at 70°C is ± 40 Pa.s. Furthermore, in the context of the present invention, a formulation is considered stable when it does not show phase separation into two different phases to the naked eye as defined for the unstable formulation.

As used herein, the term "nanoparticle" refers to a colloidal system of a solid particle with an average size less than 1 micrometer (µm), typically between 1 and 999 nanometers (nm), preferably between 50 and 500 nm, more preferably between 100 and 400 nm, still more preferably between 120 and 200 nm, still more preferably between 120 and 160 nm approximately. Depending, among other facts, on their manufacture method, nanoparticles can be subdivided into matrix nanospheres and "core-shell vesicular nanocapsules". "Matrix nanospheres" are matrix forms formed by a three dimensional network; when a nanosphere is loaded with a product of interest or active ingredient, e.g., a drug, said product of interest can be physically and uniformly dispersed in said three dimensional network. "Core-shell vesicular nanocapsules" are vesicular systems formed by an inner cavity (known as the "core") which contains the product of interest surrounded by a wall or membrane (known as the "shell"), i.e., the core-shell vesicular nanocapsules of the invention are nano-vesicular systems that exhibit a typical core-shell structure in which the product of interest or active ingredient is confined to a reservoir or within a cavity ("core") surrounded by a wall or membrane ("shell"). The skilled in the art knows that the core of the core-shell vesicular nanocapsule may contain only excipients, may contain any active ingredient as defined hereinafter (for example a compound having pharmaceutical activity or mixtures thereof with or without excipients), or may contain both excipients and said active ingredient as defined hereinafter. In both cases, due to the large specific surface of these systems, the molecules of the active ingredient may be trapped or adsorbed in the surface of the nanoparticles.

### Composition of the invention

In a first aspect, the present invention refers to a composition having a dynamic viscosity from 4 to 300 Pa.s comprising:
(i) a polyol selected from the group consisting of glycerol, propylene glycol and a combination thereof in an amount of at least 60 wt %;
(ii) a swelling polymer selected from the group consisting of celluloses and a derivative thereof or a mixture thereof in an amount of at least 1 wt %, wherein the derivative of celluloses is selected from the group consisting of a salt of carboxymethyl cellulose (CMC), ethylcellulose, hydroxyethyl cellulose, hydroxyethylmethyl cellulose, hydroxypropyl methylcellulose (HPMC) or hypromellose or a derivative thereof, hydroxypropyl cellulose (HPC), low-substituted hydroxypropyl cellulose (L-HPC), methylcellulose, and a combination thereof;
(iii) an inorganic salt comprising divalent cations selected from the group consisting of CaSO₄, CaCl₂, MgCl₂, CuSO₄, and CaCO₃ or a mixture thereof in an amount ranging from 0.01 to 5 wt %; and
(iv) a solid waxy surfactant selected from the group consisting of glyceryl monostearate, sorbitan monostearate, cetostearyl alcohol and combinations thereof
wherein said composition does not comprise more than 3.5 wt % of water, wherein wt % is the weight of each component relative to the total weight of the composition and wherein the ratio component (ii)/component (iii) is from 4 to 250; and wherein the dynamic viscosity is measured at a temperature of 19 ± 2 °C and at 12 rpm as rotational speed.

This composition has viscosity values ranging from 4 to 300 Pa.s, which are stable over the periods of time described in the examples below.

Component (i) of the composition of the invention is in an amount of at least 60 wt %, at least 65 wt %, at least 70 wt %, at least 75 wt %, at least 80 wt %, at least 85 wt %, at least 90 wt %, at least 95 wt%.

Component (ii) of the composition of the invention is in an amount of at least 1 wt%, at least 5 wt%, at least 10 wt%, at least 15 wt%, at least 20 wt%, at least 25 wt%, at least 30 wt%. In a preferred embodiment, component (ii) is in an amount ranging from 1 wt % to 30 wt%, preferably from 5 wt% to 30 wt%.

Component (iii) of the composition of the invention is in amount ranging from 0.01 to 5 wt%, preferably from 0.5 to 5 wt%, preferably from 1 to 5 wt%, preferably from 1.5 to 5 wt%, more preferably from 2 to 5 wt%, even more preferably from 2.5 to 5 wt%, most preferably from 3 to 5 wt%.

Component (iv) may be in an amount of at least 0.1 wt%, at least 0.5 wt%, at least 1 wt%, at least 2 wt%, at least 3 wt%, at least 4 wt%, at least 5 wt%, at least 10 wt%, at least 15 wt%, at least 20 wt%, at least 25 wt%, at least 30 wt%, at least 35 wt%, at least 36 wt%, at least 37 wt%, at least 38 wt% or at least 39 wt%.

In a preferred embodiment, the composition comprises:
(i) 60 to 95 wt % of a polyol selected from the group consisting of glycerol, propylene glycol and a combination thereof;
(ii) 1 to 30 wt % of a swelling polymer selected from the group consisting of celluloses and a derivative thereof or a mixture thereof, wherein the derivative of celluloses is selected from the group consisting of a salt of carboxymethyl cellulose (CMC), ethylcellulose, hydroxyethyl cellulose, hydroxyethylmethyl cellulose, hydroxypropyl methylcellulose (HPMC) or hypromellose or a derivative thereof, hydroxypropyl cellulose (HPC), low-substituted hydroxypropyl cellulose (L-HPC), methylcellulose, and a combination thereof;
(iii) 0.01 to 5 wt % of an inorganic salt comprising divalent cations selected from the group consisting of CaSO₄, CaCl₂, MgCl₂, CuSO₄, and CaCO₃ or a mixture thereof; and
(iv) 0.1 to 10 wt % of a solid waxy surfactant selected from the group consisting of glyceryl monostearate, sorbitan monostearate, cetostearyl alcohol and combinations thereof.

Component (i) of the composition of the invention is selected from the group consisting of glycerol, propylene glycol and a combination thereof.

Component (ii) of the composition of the invention is selected from the group consisting of celluloses, a salt of carboxymethyl cellulose (CMC), ethylcellulose, hydroxyethyl cellulose, hydroxyethylmethyl cellulose, hydroxypropyl methylcellulose (HPMC) or hypromellose or a derivative thereof, hydroxypropyl cellulose (HPC), low-substituted hydroxypropyl cellulose (L-HPC), methylcellulose, and a combination thereof. In a more preferred embodiment, component (ii) of the composition of the invention is selected from the group consisting of calcium CMC, sodium CMC, ethylcellulose, hydroxyethyl cellulose, hydroxyethylmethyl cellulose, hypromellose, hypromellose acetate succinate, hypromellose phthalate, hydroxypropyl cellulose (HPC), low-substituted hydroxypropyl cellulose (L-HPC), methylcellulose, and a combination thereof. In an even more preferred embodiment, component (ii) of the composition of the invention is selected from the group consisting of a salt of carboxymethyl cellulose (CMC), hydroxypropyl methylcellulose (HPMC), hydroxypropyl cellulose (HPC) and a combination thereof. In another preferred embodiment component (ii) is a salt of carboxymethylcellulose (CMC), more preferably sodium CMC. In another preferred embodiment, the salt of CMC is in an amount ranging from 1 to 30 wt%, preferably 5 to 30 wt%, more preferably 10 to 30 wt%, more preferably 15 to 30 wt%, even more preferably 15 to 25 wt%.

Component (iii) is selected from the group consisting of CaSO₄, CaCl₂, MgCl₂, CuSO₄, and CaCO₃ or a mixture thereof. In a more preferred embodiment is CaSO₄.

The surfactant can be dispersed or emulsified within the composition.

Component (iv) is selected from the group consisting of glyceryl monostearate, sorbitan monostearate, cetostearyl alcohol and combinations thereof.

The inventors have found that for compositions with less than 15 % w/w of CMC and waxy surfactant concentration from 1 to 3 % w/w, no phase separation was observed. Therefore, in a preferred embodiment, the composition comprises less than 15% w/w of CMC and a solid waxy surfactant ranging from 1 to 3% w/w.

The composition of the invention may further comprise a compound selected from the group consisting of a thickener, a polysaccharide different from component (ii), a surfactant different from component (iv), an adhesive polymer, a fat, and a combination thereof.

When the composition of the invention comprises a thickener agent, this may be selected from the group consisting of copovidone, carbomer, polyethylene oxide, polyethylene glycol, a polysaccharide different from celluloses or a derivative thereof, silica and combinations thereof; preferably selected from the group consisting of xanthan gum, copovidone and combinations thereof. Preferably, xanthan gum is in an amount ranging from 0.1 to 2 wt%. Preferably, the thickener agent is present in an amount ranging from 0.1 to 5 wt %.

The composition may also contain polysaccharides different from component (ii) that are suitable for improving organoleptic characteristics of the formulation.

When the composition of the invention comprises a polysaccharide different from component (ii), this may be selected from the group consisting of sodium alginate, sodium carragenate, pectine, guar gum, xanthan gum, acacia gum, gellan gum, tragacanth gum, agar-agar, starch and combinations thereof. Preferably, said polysaccharide different from component (ii) is in an amount from 0.1 to 10 wt%.

When the composition of the invention comprises a surfactant different from component (iv), this may be selected from the group consisting of polyoxyl 5 castor oil, polyoxyl 9 castor oil, polyoxyl 35 castor oil, polyoxyl 4 lauryl ether, polyoxyethylene 20 sorbitan, polyoxyethylene (4) sorbitan monolaurate, polyoxyethylene 20 sorbitan monolaurate, polyoxyethylene 20 sorbitan monopalmitate, polyoxyethylene 20 sorbitan monostearate, polyoxyethylene (5) sorbitan monooleate, polyoxyethylene 20 sorbitan monooleate, polyoxyethylene 20 sorbitan trioleate, polyoxyethylene 20 sorbitan monoisostearate, sorbitan monoisostearate, sorbitan monolaurate, sorbitan monooleate, sorbitan sesquioleate, sorbitan trioleate, glyceryl monooleate, linoleoyl polyoxylglycerides, oleoyl polyoxylglycerides, polyethylene glycol 200, polyethylene glycol 300, polyethylene glycol 400, polyethylene glycol 600 and combinations thereof.

When the composition of the invention comprises an adhesive polymer, this is a polymer capable of forming *in situ* nanoparticles when in contact with an aqueous medium. Said polymer may be selected from the group consisting of half esters of poly(methyl vinyl ether-co-maleic anhydride) (PVM/MA) copolymers, poly(methylvinyl ether/maleic acid), zein, polymethacrylates and a combination thereof.

Preferably, the PVM/MA copolymers are selected from the group consisting of butyl ester of PVM/MA copolymer, isopropyl ester of PVM/MA copolymer, and ethyl ester of PVM/MA copolymer.

Preferably, polymethacrylates are selected from the group consisting of poly(butyl) methacrylate, (2-dimethylaminoethyl) methacrylate, methyl methacrylate, poly(ethyl acrylate, methyl methacrylate), poly(methacrylic acid, methyl methacrylate), poly(methacrylic acid, ethyl acrylate), poly(methyl acrylate, methyl methacrylate, methacrylic acid), and poly(ethyl acrylate, methyl methacrylate, trimethylammonioethyl methacrylate chloride).

As used herein, the term "adhesive polymer" relates to a polymer that after being dissolved in compound (i) is capable of forming *in situ* nanoparticles when in contact with an aqueous medium.

When the composition of the invention comprises a fat, this may be selected from the group consisting of linseed oil, wheat germ oil, medium chain triglycerides, olive oil, paraffin, lanolin, *Vitis vinifera* seed oil and a combination thereof. Preferably, the fat is in an amount ranging from 0.5 to 30 wt %, preferably from 0.5 to 20 wt %, preferably from 0.5 to 15 wt %, preferably from 0.5 to 10 wt %, more preferably from 0.5 to 8 wt %, more preferably from 0.5 to 5 wt %, even more preferably from 0.5 to 1 wt %.

In an embodiment the composition of the invention does not contain a fat, preferably does not contain an oil. In another embodiment, the composition of the invention contains less than 40% w/w of oil or fat, preferably less than 35% w/w, more preferably less than 30% w/w, more preferably less than 25% w/w, more preferably less than 20% w/w, more preferably less than 15% w/w, more preferably less than 10% w/w, even more preferably less than 9% w/w, less than 8% w/w, less than 7% w/w, less than 6% w/w, less than 5% w/w, less than 4% w/w, less than 3% w/w, less than 2% w/w, less than 1% w/w, less than 0.5% w/w, less than 0.1 % w/w, more preferably 0% w/w. In an embodiment the fat or oil comprises 0 to 10 wt% of the composition.

Thus, the base composition of the present invention may optionally incorporate additional components. In a preferred embodiment, the composition comprises:
(i) 60 to 90 wt % of glycerol;
(ii) 5 to 25 wt % of sodium carboxymethylcellulose;
(iii) 0.1 to 2.5 wt % of calcium sulphate;
(iv) 0.1 to 7 wt % of glyceryl monostearate;
(v) 0 to 2 wt % of xanthan gum;
(vi) 0 to 3 wt % of copovidone;
(vii) 0 to 10 wt % of sodium alginate; and
(viii) 0 to 10 wt % of *Vitis vinifera* seed oil.

Further, the composition of the invention may comprise at least an active ingredient.

As used herein, the term "active ingredient" or "Al" refers to any compound selected in order to achieve a desired effect on a selected mucous membrane. Particularly, the active ingredient is suitable for topical or oral use. Illustrative, non-limiting examples of an "Al" according to the present invention include antibiotics, antimycotics, enzymes, antiprotozoals, antivirals, anti-inflammatories, antihistamines, antiperiodontosis agents, antifungals, antibacterials, whitening agents, disinfectants, analgesics, anesthetics, wound healing agents, anti-nauseants, chemotherapeutics, immunosuppressive agents, immunomodulators, antiseptics, mucolytics, antitussives, decongestants, calcium absorption and regulating agents, hormones, vaccines, biological agents, personal care products, natural plant extracts, vitamins, omega 3- oils, amino acids, peptides and combinations thereof.

In a particular embodiment, the active ingredient is selected from the group consisting of miconazole, carbenoxolone, dexamethasone, triamcinolone, lidocaine, clobetasol, dipotassium glycyrrhizate, dexpanthenol, allantoin, chlorhexidine digluconate and mixtures thereof.

### Uses of the composition of the invention

In a second aspect, the invention relates to the composition of the invention further comprising at least an active ingredient for use as a medicament.

In a third aspect, the invention relates to the composition of the invention further comprising at least an active ingredient for use in the prevention and/or treatment of adisease selected from the group consisting of:
(a) a buccal disease selected from the group consisting of xerostomia, keratosis, desquamative stomatitis (or gingivitis), candidiasis, pericoronitis, erythroplasia, burning mouth syndrome, cancer sores, cold sores, geographic tongue, leukoplakia, lichen planus, erythema multiforme, drug eruptions, halitosis, periodontitis, pemphigus vulgaris, Sjögren's syndrome, gingivitis, ulceration, recurrent aphtous stomatitis and malignant ulcers;
(b) a gastric/gastroesophageal disease selected from the group consisting of gastroesophageal reflux and esophagitis;
(c) an anal disease that is the haemorrhoidal disease; and
(d) a vaginal disease selected from the group consisting of bacterial vaginosis, vaginal yeast infection, vaginitis, chlamydia, gonorrhea, genital herpes, and genital warts.

Exemplary vaginal yeast infections can be, without limitation, candidiasis.

In another aspect, the invention relates to the use of the composition of the invention as an adhesive for dentures.

### Method for preparing the composition of the invention

In another aspect, the present invention provides a method for preparing the composition described above comprising:
(a) dispersing component (iii) and component (iv) within component (i); and
(b) dispersing component (ii) in the mixture resulting from the previous step.

The person skilled in the art knows techniques for dispersing said components.

Depending on different factors such as the kind of solid waxy surfactant, its concentration, or the rest of components of the formulation, heating may be needed to obtain the composition of the invention. Preferably, heating is needed when the concentration of the solid waxy surfactant is higher than 0.5 wt%.

Optionally, the method for preparing the composition of the invention further comprises at least two additional steps between steps (a) and (b), said steps being:
I. heating the mixture resulting from step (a) at least at the melting temperature of component (iv) and not exceeding a temperature of 70°C; and
II. emulsifying the mixture resulting from step (I) while cooling at least to 40°C for the obtention of a composition according to the invention.

In step I the mixture is heated at least at a temperature of 35 °C, at least at 45 °C, at least at 50°C, at least at 55 °C, at least at 60 °C, at least at 65 °C and not exceeding a temperature of 70°C.

In step II the mixture is emulsified for example by stirring the mixture resulting from step I with a paddle stirrer at 2000 rpm. Said emulsification is made while cooling at least to 40°C, preferably at least to 35 °C.

When the composition of the invention optionally comprises a thickener and/or a polysaccharide different from component (ii), and/or a surfactant different from component (iv), and/or an adhesive polymer and/or a fat, the method for preparing the composition further comprises the steps of dispersing, dissolving or swelling a thickener and/or a polysaccharide different from component (ii), and/or a surfactant different from component (iv) and/or an adhesive polymer and/or a fat within the polyol or a mixture thereof in an amount of at least 60 wt % in step (a) or adding said thickener and/or polysaccharide different from component (ii), and/or surfactant different from component (iv), and/or adhesive polymer and/or fat after step (b).

When the composition of the invention optionally comprises an active ingredient, the method for preparing the composition further comprises the step of incorporating the active ingredient before, during or after step (b).

### Method for obtaining a nanoparticle composition

It is known that contacting a composition containing an adhesive polymer dissolved in compound (i), wherein said adhesive polymer is selected from the group consisting of half esters of poly(methyl vinyl ether-co-maleic anhydride) (PVM/MA) copolymers, poly(methylvinyl ether/maleic acid), zein and polymethacrylates, with an aqueous medium allows obtaining *in situ* nanoparticles. This has been disclosed, for example, in WO02013120856 A1 or WO2012140252A1.

Therefore, in another aspect the invention relates to a method for obtaining a nanoparticle composition comprising contacting a composition according to the invention that contains an adhesive polymer dissolved in compound (i), with an aqueous medium, wherein said adhesive polymer is selected from the group consisting of half esters of poly(methyl vinyl ether-co-maleic anhydride) (PVM/MA) copolymers, poly(methylvinyl ether/maleic acid), zein and polymethacrylates.

Preferably, the aqueous medium is a biological fluid, more preferably salivary fluid.

The invention is described below by means of several examples which do not limit, but rather illustrate the invention.

### EXAMPLES

### Example 1

Different formulations were prepared by dispersing the swelling polymer (a cellulose derivative such as sodium CMC, HPMC or HPC) in a polyol solvent (glycerol) with or without divalent salts (CaSO₄, CaCl₂, MgCl₂, CuSO₄ or CaCO₃) and waxy surfactants (glyceryl monostearate, Span^{®} 60 or cetostearyl alcohol). When no salts and surfactants were used, the swelling polymer was mixed at room temperature with the polyol solvent. When salts were used, a previous dispersion of the salt in the polyol was prepared and then the swelling polymer was added to the mixture. When waxy surfactants were used, the salt and the waxy surfactant were dispersed in the polyol, then the mixture was heated to 55-60 °C and emulsified in the polyol solvent when cooling to 40°C. Finally, the cellulose derivative was added to the mixture after cooling.

The viscosity of the compositions was evaluated just after their preparation, after one day of storage at 40 °C and after one week of storage at 40 °C. The viscosity of each formulation was measured with a digital viscometer (Viscotech Hispania, V1-R) by controlling different critical parameters: sample temperature, spindle characteristics and rpm. Table 1 shows the results.

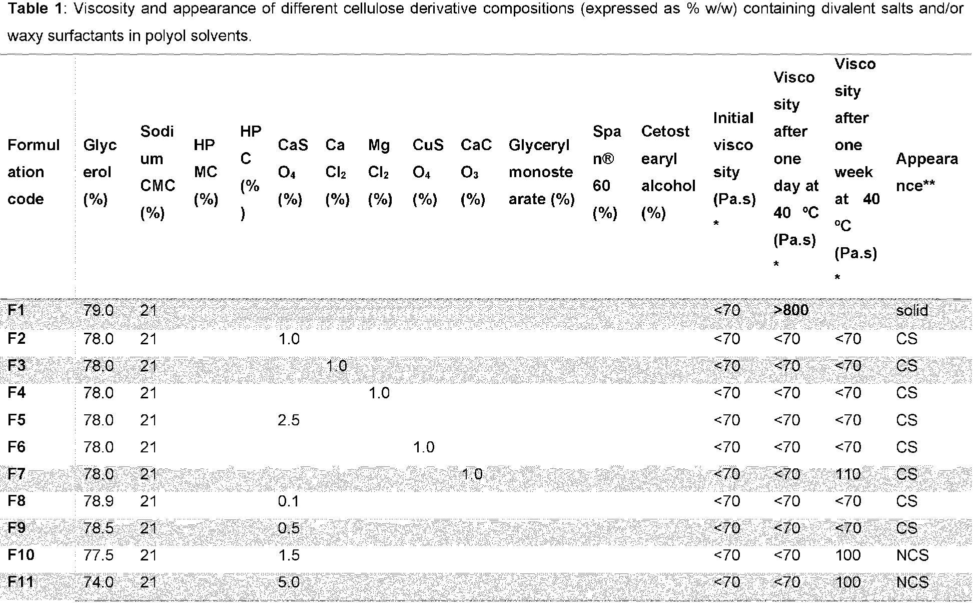

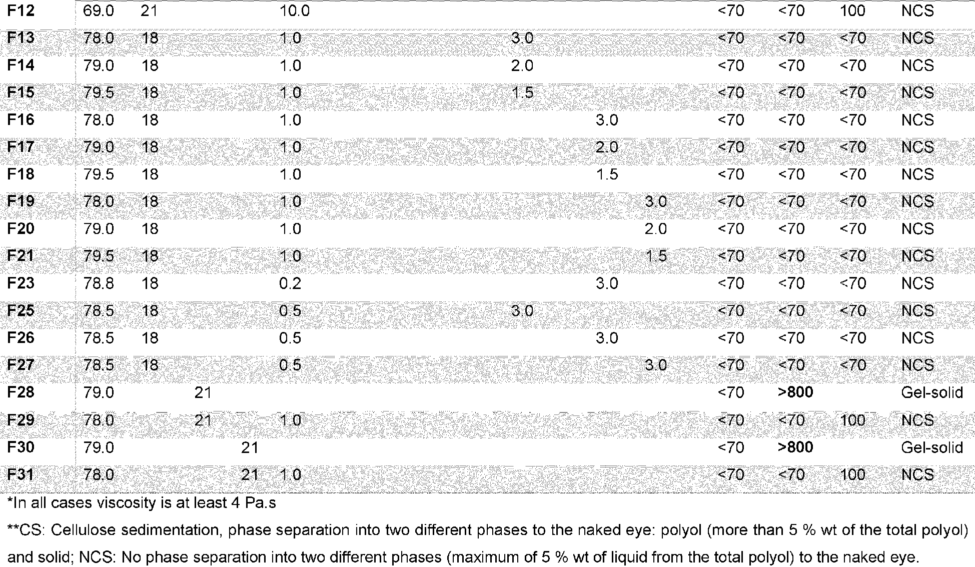

Compositions suffering cellulose sedimentation (CS) are not suitable for use. However, compositions having no sedimentation or low sedimentation effect (≤ 5 % wt of liquid from the total polyol) without phase separation to the naked eye, can be used.

Results demonstrated that when cellulose derivatives such as sodium carboxymethyl cellulose (CMC), hydroxypropyl methylcellulose (HPMC) or hydroxypropyl cellulose (HPC) were dispersed in a polyol solvent (glycerol) without divalent salts, the viscosity of the mixture dramatically increased over time due to the swelling of the polymer, so the bioadhesive properties of the swelling polymer decreased proportionally to the viscosity change. Finally, after several days at 40 °C, the mixture became solid (Formulations F1, F28 and F30). However, when different divalent salts were dispersed in the polyol solvent before the addition of the cellulose derivative, the viscosity of the mixture does not exceed 110 Pa.s thus conserving their adhesive properties. However, it was surprisingly observed that this effect takes place only when the concentration of the divalent salt was in the range of 0.1 to 5 % w/w (for formulations with swelling polymer concentration of 21% w/w). When the divalent salt concentration was higher than 5 % w/w (for formulations with swelling polymer concentration of 21 % w/w), the polymer swelling inhibition phenomena was reduced and formulations became unstable over time. For example, F12 containing 10 % w/w of salt is stable after one week at 40°C, but it becomes unstable over time due to an increase of its viscosity over 110 Pa.s (data not shown). Therefore, when the ratio swelling polymer /divalent salt concentration is lower than 4, the formulation is not stable. Thus, the optimum ratio is from 4 to 250. In these conditions, cellulose derivatives do not swell in the polyol solvent but are only dispersed, and hence maintain their adhesive properties over time.

On the other hand, when the swelling agent used was CMC (see F2), a clear polymer sedimentation effect was observed in the polyol solvent. This effect was less evident when HPMC or HPC were used instead of CMC (see F29 and F31). Nevertheless, when a solid waxy surfactant was dispersed or emulsified in the mixture before the addition of the swelling agent, the sedimentation effect significantly decreased. Moreover, the higher the amount of waxy surfactant was used, the less sedimentation effect was found (see also Table 2). In fact, for compositions with less than 15 % w/w of CMC and waxy surfactant concentration from 1 to 3 % w/w, no phase separation was observed.

### Example 2

Different formulations were prepared with calcium sulphate as an inorganic salt comprising divalent cations, a polyol or a combination of polyols (glycerol and/or polyetilenglycol), a solid waxy surfactant (glyceryl monostearate or Span^{®} 60), a derivative of cellulose as swelling polymer (CMC), a polysaccharide different from cellulose or a derivative thereof acting as swelling agent (sodium alginate), and at least a thickener (xanthan gum and/or copovidone). The viscosity of those formulations was evaluated in the moment of manufacturing and over time, in order to test their stability. Table 2 shows the composition of the different formulations and the viscosity results obtained. As it can be seen, in all cases, regardless of the thickener, the second swelling agent and the waxy surfactant used, formulations remained stable over time (when the formulation has an initial viscosity < 70 Pa.s if it does not increase over 110 Pa.s over time it is considered stable; and when the formulation has an initial viscosity > 70 Pa.s if the variation of the viscosity is not higher than ± 40 Pa.s over time it is considered stable). If the inorganic salt having divalent cations was not present, the formulation would have an abrupt increase in viscosity (> 800 Pa.s) in several hours or days. These excipients (thickener, second swelling agent, waxy surfactant, etc) can affect the initial viscosity of the formulation and the adhesive properties of the final product, but they do not interfere with the viscosity variation over time.

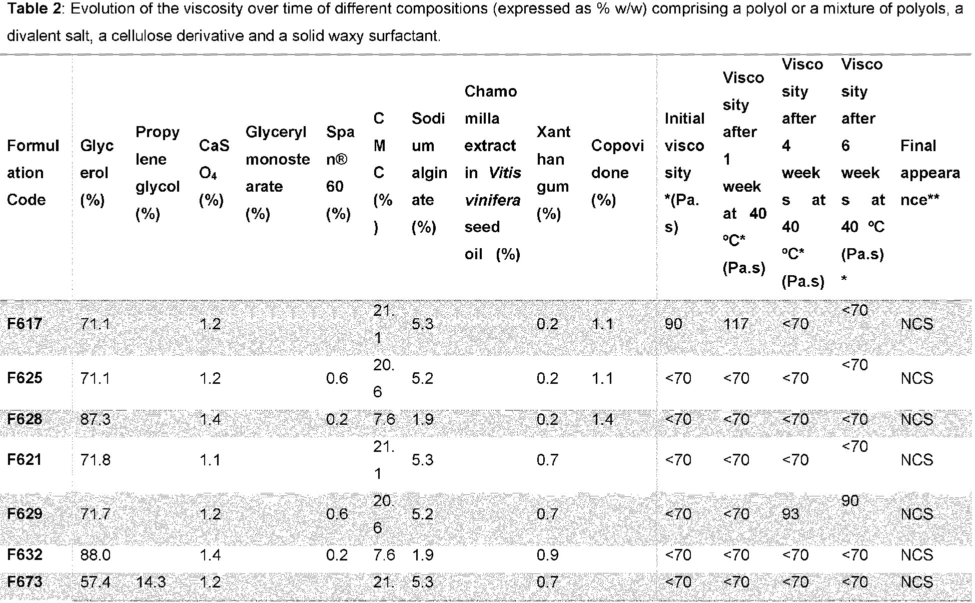

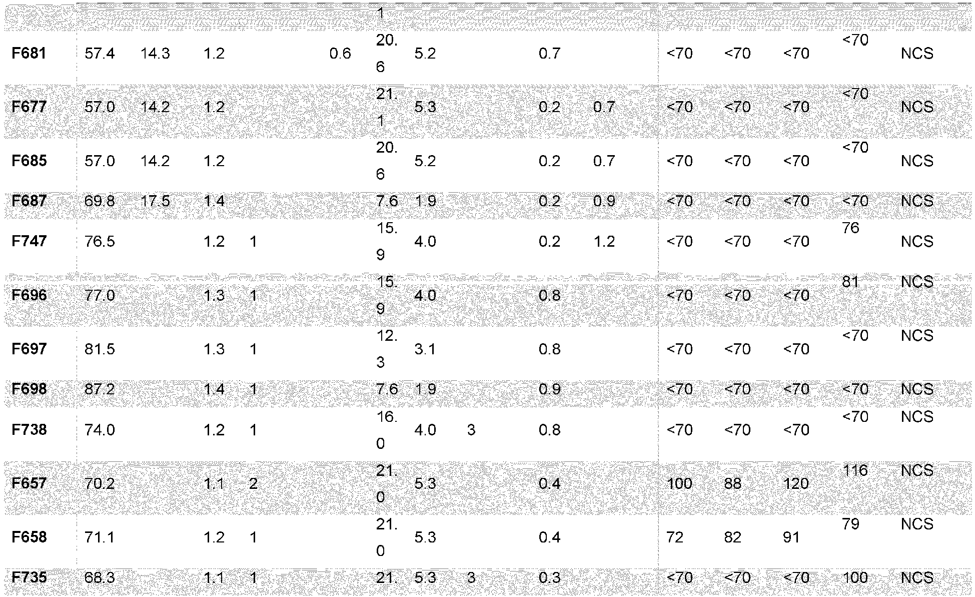

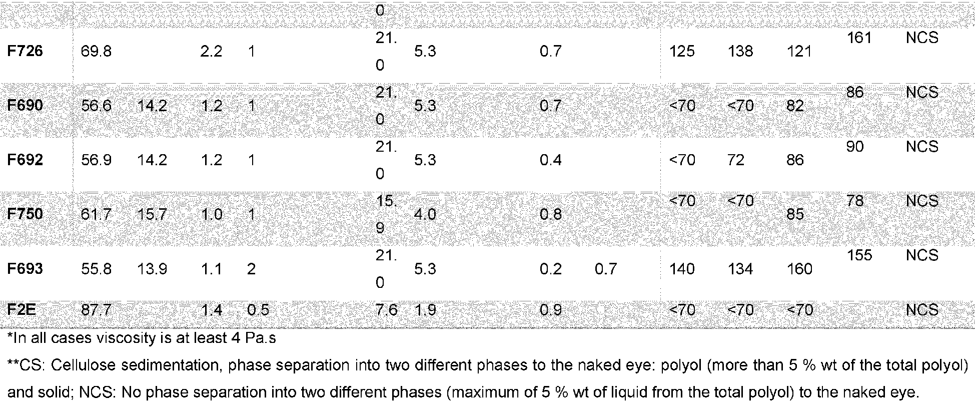

### Example 3

In order to evaluate the impact of the different components on the adhesive properties of the product, a mucosal adhesion study was conducted.

For that purpose, a synthetic mucosal base was prepared by weighing 25 g of type III pig mucin, 20 g of carrageenan and 700 mL of water in a beaker, heating the mixture at 70 °C for 15 min and homogenizing it. Finally, before cooling, 30 mL of the mixture was added to beakers with 6 cm diameter and left to solidify at room temperature.

Each plate was used to test the adhesive properties of one formulation. For that purpose, in the same beaker, 4 different spots of formulation were deposited. Each formulation spot consisted of approximately 0.1 g of product.

Just after formulation deposition, each plate was filled with 60 mL of phosphate buffer saline (pH 7.4) and incorporated in an orbital incubator set at 37 °C and 150 rpm. The time at which each formulation spot was released from the mucin was registered. The average time at which the four spots were released was considered the adhesion time.

For the comparative study, tree different plates were used per formulation (each plate containing 4 formulation spots). The average adhesion time obtained for each plate was calculated for each specific formulation and compared with the rest of compositions.

Those formulations that presented viscosity increments over time were tested at the moment of preparation and also several days afterwards, when the viscosity was significantly higher, in order to confirm that the adhesive properties diminish proportionally to the viscosity increase, which was due to the polymer swelling. Additionally, formulations without viscosity increments over time were also tested at different times in order to confirm that the adhesive properties remained unaltered. All of the formulations were stored at 40 °C.

Finally, the study was also conducted with several commercial products such as Orabase^{®} (commercialized by Guinama), Sanodin^{®} (carbenoxolone formulation in Orabase^{®} commercialized by ERN laboratories) and different commercial gels based on hyaluronic acid as a swelling agent (Aftamed gel^{®} by GUM laboratories, Aloclair plus gel^{®} by Sinclair laboratories and Aftum gel oral^{®} by Viñas laboratories). Therefore, the adhesion times of such formulations were calculated by this method and compared with the formulations designed by the inventors. This study was not repeated at different times because it was considered that for commercial products, the adhesive properties keep constant over time.

The composition of the commercial products is as follows:
- Orabase^{®} contains mineral oil/paraffinum (80-90%), polyethylene glycol (2-7%), sodium carboxymethylcellulose 1500-4500 (2-7%), gelatin bloom 140/160 type A (2-7%), and pectin (2-7%).
- Sanodin^{®} (20 mg/g) contains disodium carbenoxolone, macrogol 400, liquid paraffin,and karaya gum.
- Aftamed gel^{®} contains sodium hyaluronate salt.
- Aloclair plus gel^{®} contains water, copovidone, maltodextrin, propylenglycol, PEG-40 hydrogenated castor oil, xanthan gum, potassium sorbate, sodium sorbate, sodium hyaluronate, aroma, benzalkonium chloride, disodium EDTA, sodium saccharin, potassium glycirrhizate and *Aloe barbadensis.*
- Aftum gel oral^{®} contains sodium hyaluronate salt.

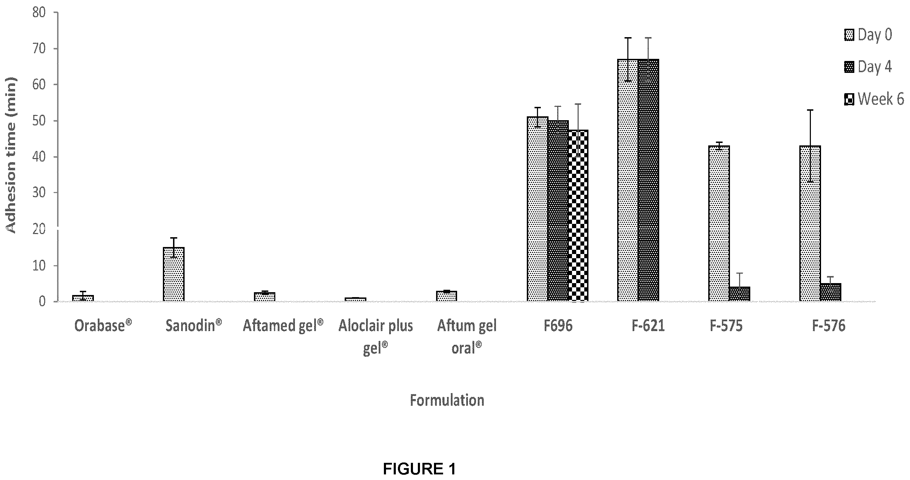

Table 3 shows the composition of the tested formulations and their viscosity and Figure 1 shows the results obtained in each case. As it can be seen, the initial adhesive properties of the gel formulations of the invention were significantly higher than that of the commercial products. Additionally, it was confirmed that when no divalent salts were present in the formulation (F575 and F576), the initial adhesive properties of the product decreased significantly over time while the viscosity increased dramatically.

Finally, for formulations with divalent salts (calcium sulphate in this case), the viscosity and the adhesive properties of the product were clearly higher than that of the commercial products and also consistent over time. However, it was observed that when the formulation did not contain waxy surfactants (F621) the amount of formulation that remained adhered over time was significantly lower than the amount of formulation adhered when the waxy surfactant was present in the formulation (F696). In fact, the 50 % of the deposited formulation (F621) was unadhered or dissolved during the first 5 minutes of the study while the rest of the product remained adhered; and in the case of F696, most of the product was adhered during the first minutes of the study and remained adhered to the artificial mucosa for all the adhesion time. This is due to the high hydrophilic properties of the F621 product, that allow the rapid penetration of high amounts of PBS which favor the initial adhesion, but swell excessive amount of swelling polymer. Therefore, the product that contains the waxy surfactant (F-696), despite being hydrophilic, limits the amount of PBS that can rapidly penetrate into the composition to just the necessary amount of PBS to hydrate the swelling polymer without dissolving the product, therefore achieving maximum residence times.

### Example 4

Formulations F696, F690 and F750 (see Table 2) were used as base to incorporate different active pharmaceutical ingredients (miconazole, carbenoxolone, dexametasone, triamcinolone, lidocaine, clobetasol, dexpanthenol, allantoin, dipotassium glycyrrhizate, chlorhexidine digluconate) as model drugs. The viscosity and macroscopic characteristics of the resulting formulations were registered overtime. Table 4 shows the composition of the different formulations and results obtained.

As it can be seen, all the studied formulations were stable for at least six weeks at 40°C. Therefore, the designed formulations of the invention are versatile for the incorporation of both hydrophilic and lipophilic ingredients with different physic-chemical properties.

### Example 5

In order to evaluate the salivary levels of hydrophobic drugs administered in the formulations of the invention in comparison with its original commercial vehicles, a study was conducted using miconazole as reference hydrophobic molecule. Therefore, the drug was dispersed in the formulation F696 to obtain a 2 % miconazole formulation. The final composition of the miconazole formulation according to the invention is shown in Table 5.

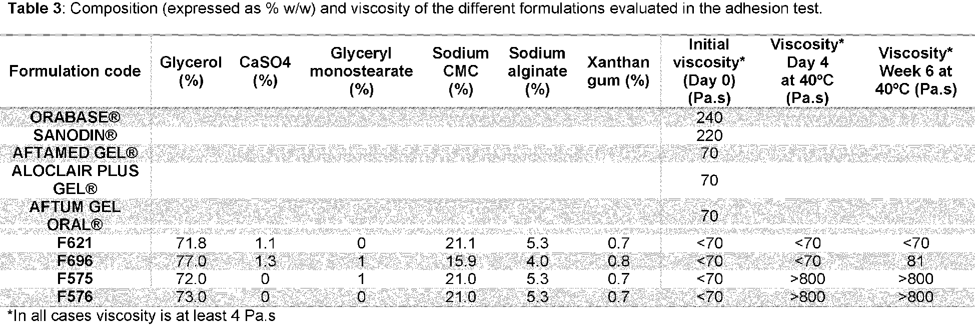

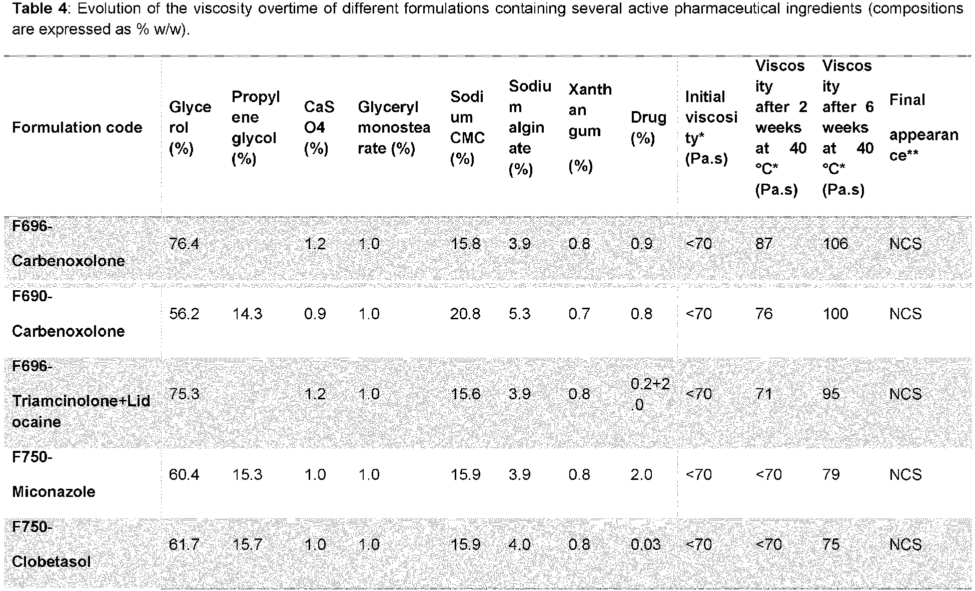

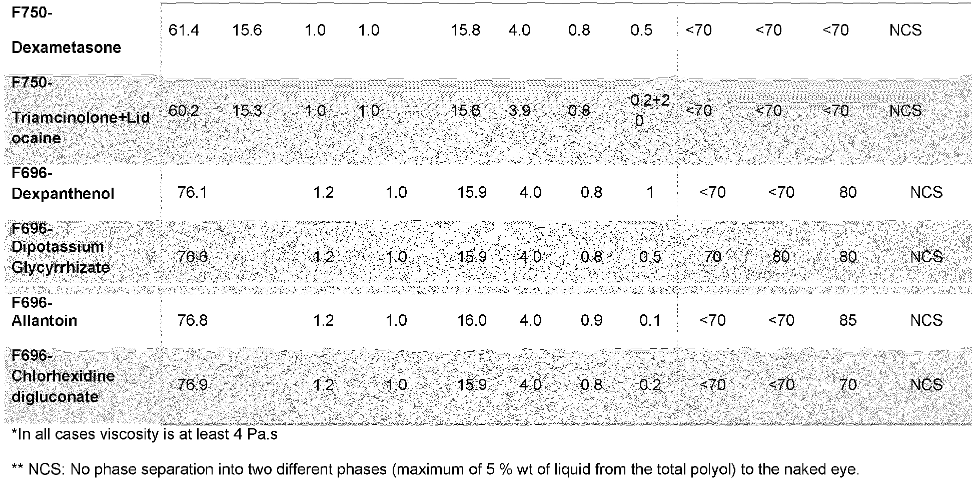

**Table 5. Composition of a formulation according to the invention containing miconazole**

| **F696** | **% (w/w)** |
|---|---|
| Glycerol | 72.94 |
| Dexpanthenol | 1.04 |
| Sodium benzoate | 0.05 |
| Butylated hydroxytoluene | 0.11 |
| Saccharine | 0.31 |
| Benzalkonium chloride | 0.11 |
| Potassium glycyrrhizate | 0.54 |
| CaSO₄ | 1.22 |
| Xanthan gum | 0.77 |
| Glyceryl monostearate | 0.97 |
| CMC | 15.55 |
| Sodium alginate | 3.97 |
| Peppermint | 0.20 |
| Miconazole | 2.01 |

1 g of the formulation was administered to 6 different human volunteers. All of them maintained the product in the mouth for one minute and subsequently swallowed it. At different times after swallowing, 1 mL of saliva was collected from each volunteer and analyzed to determine miconazole salivary concentration. The samples were subjected to a protein precipitation extraction process: 200 µL of saliva were mixed with 200 µL of an ACN:MeOH (50:50) solution, vortexed for 30 seconds and centrifuged at 15000 rpm for 10 minutes. The supernatant was filtered through 0.22 µm polytetrafluoroethylene (PTFE) membranes and afterwards injected in the HPLC under the following chromatographic conditions:
Analytical column: Zorbax Bonus-RP(150x4.6 mm), 5µm
Mobile phase: ACN: 25mM NaH₂PO₄ in a ratio 65:35
Flow: 1 mL/min
Injection volume: 20 µL
Detection wavelength: 220 nm

This procedure was conducted for both Daktarin^{®} (commercial miconazole reference product commercialized by Esteve laboratories) and the miconazole formulation of the invention.

Daktarin^{®} is composed of sodium saccharine, pregelatinized starch, polysorbate 20, orange flavour, cocoa flavour, ethanol, glycerol and distilled water.

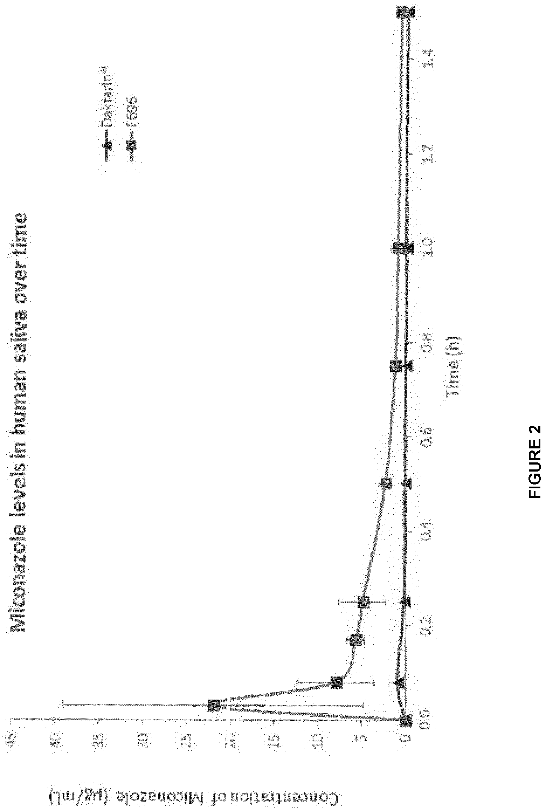

Figure 2 shows the results obtained in both cases. As it can be seen, both the drug concentration levels and the drug residence time were significantly higher for the formulations of the invention in comparison with the reference product Daktarin^{®}.

**Table 6. Area under the curve calculated from miconazole salivary levels found in saliva over time after buccal administration.**

| **Formulation** | **Daktarin^{®}** | **F696** |
|---|---|---|
| **AUC (µg·h/mL) (mean ± SD)** | 0.3 ± 0.2 | 5.1 ± 1.23** |

| | | |
|---|---|---|
| ** p< 0.01, (n=6) | | |

### Example 6

In order to evaluate the *in vitro* release and the adhesive properties of hydrophilic drugs administered in the formulations of the invention in comparison with its original commercial vehicles, a study was conducted using carbenoxolone as reference hydrophilic molecule.

Therefore, the drug was dispersed in the formulation F696 to obtain a 2 % carbenoxolone formulation. Additional excipients were used in order to optimize the organoleptic properties of the final product. The final composition of the carbenoxolone formulation of the invention is showed in Table 7.

**Table 7. Composition of a formulation according to the invention containing carbenoxolone**

| F696 | % (w/w) |
|---|---|
| Glycerol | 73.32 |
| Dexpanthenol | 0.87 |
| Sodium benzoate | 0.05 |
| Butylated hydroxytoluene | 0.12 |
| Saccharine | 0.33 |
| Benzalkonium chloride | 0.11 |
| Potassium glycyrrhizate | 0.54 |
| CaSO₄ | 1.23 |
| Xanthan gum | 0.77 |
| Glyceryl monostearate | 0.98 |
| CMC | 15.55 |
| Sodium alginate | 3.97 |
| Carbenoxolone | 2.16 |

For that purpose, a synthetic mucosal base previously described in Example 3 was prepared. In this case, before cooling, 0.5 mL of the mixture was added to a tube cap with 1 cm diameter and left to solidify at room temperature.

For the release-adhesion study, 0.1 g of formulation (2 mg of carbenoxolone) were deposited into the synthetic mucosa, which was then placed into the tube containing 14 mL of PBS, and set for 4 hours at 37°C under orbital agitation (100 rpm). At different time points, synthetic mucosae with the adhered formulation were collected to determine the amount of carbenoxolone adhered or released through the synthetic mucosa.

The samples were subjected to an extraction process: synthetic mucosae were mixed with 5 mL of highly purified water and vortexed for 30 min. After that, 5 mL of an ACN solution were added and the mixture was vortexed for 30min and centrifuged at 15000 rpm for 10 minutes. The supernatant was filtered through 0.45 µm PTFE membranes and afterwards injected in the HPLC under the following chromatographic conditions:
Analytical column: Zorbax SB-C18 (150x4.6mm), 5µm
Mobile phase: ACN: 50mM H₃PO₄ in a ratio 70:30
Flow: 1.5 mL/min
Injection volume: 20 µL
Column temperature: 25 °C
Detection wavelength: 254 nm

This procedure was conducted for both Sanodin^{®} (commercial carbenoxolone reference product in Orabase^{®} commercialized by ERN laboratories) and the carbenoxolone formulation of the invention.

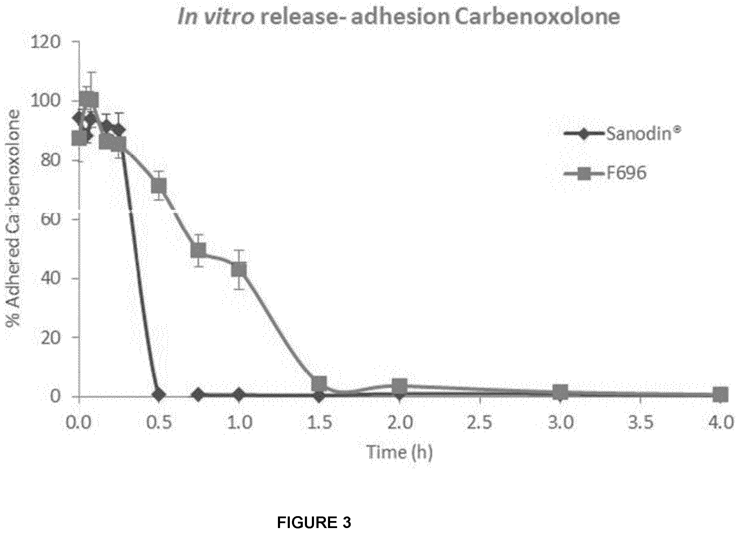

Figure 3 shows the results obtained in both cases. As it can be seen, both the percentage of retained drug and the drug residence time were higher for the formulation according to the invention in comparison with the reference product Sanodin^{®}.

Considering that both formulations are designed to maximize the residence time of the drug in the site of application and minimize the drug released over time, it was considered that the formulation of the invention shows improving properties with respect to the commercial reference product Sanodin ^{®}.

### Example 7

In order to evaluate the bioadhesive properties of the product in oesophageal mucosa, a retention *ex vivo* study in horse oesophagi was carried out.

Horse oesophagi were collected from slaughtered animals, the outer muscle layer was removed exposing the oesophagus and they were cut to ensure uniform tubes of 16 cm. An entry and an exit tube were placed on the oesophagus ends. This system was assembled onto a ramp and covered with aluminium foil to reduce moisture loss. Firstly, the oesophagi were washed by flushing PBS through the entry port. Secondly, a continuous flow of 1ml/min of PBS was pumping in during all the experiment, simulating saliva flow. Finally, 1 g of the products assayed was introduced into the lumen through the dosing port and a roller was run down the length of esophagus manually to mimic "peristaltic waves".

After 1 or 2 hours, oesophagi were longitudinally cut and the product retained was observed in different regions of the mucosa. To improve the visualization of the adhered formulation, a blue dye, indigo carmine, was used at 0.1%.

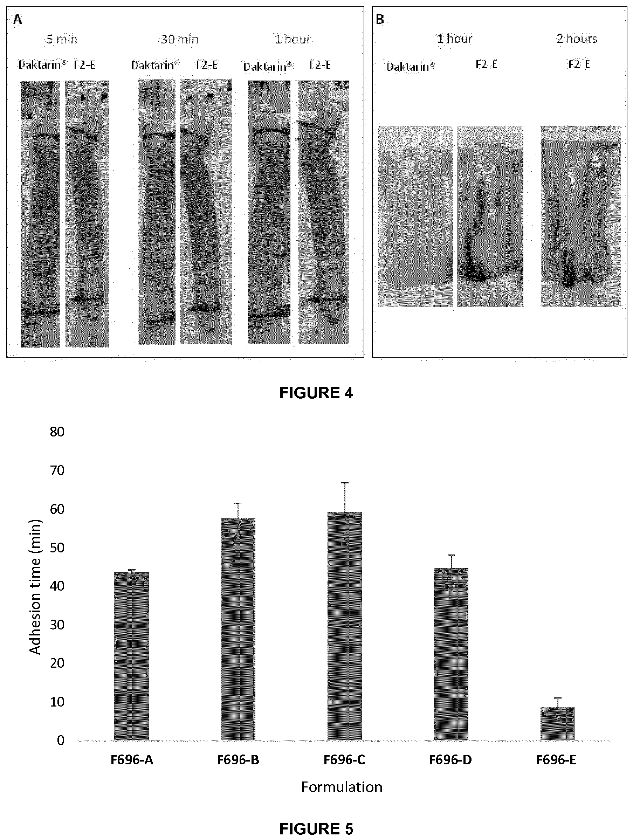

As Figure 4-A shows, five minutes after their administration, both formulations Daktarin^{®} (commercial product) and F2-E (formulation according to the invention; see last composition of Table 2) displayed a similar distribution (dark shadow inside oesophagus due to the blue dye). However, F2-E was able to remain longer that Daktarin^{®}. In order to confirm this, oesophagi were cut (Figure 4-B). Even after 2 hours, a higher amount of the formulation was adhered to oesophagus, covering a larger surface.

### Example 8

In order to establish the viscosity limits of the formulations, a mucosal adhesion study was conducted.

For that purpose, the experiment described in example 3 with the formulation F696 at different viscosities was performed.

For the comparative study, the formulations were subjected to aggressive conditions (70°C for 1, 2 and 4h). Table 8 shows the composition of the tested formulations and their respective viscosities and Figure 5 shows the change in adhesion time depending on the viscosity of each formulation. As it can be seen, the adhesive properties of the formulations were maintained between 35-60 min in those formulations where the viscosity ranged between 4-300 Pa. Higher viscosities caused a loss of adhesion (F696-E).

### Example 9

Different formulations were prepared by dispersing the swelling polymer in a polyol with oxides (ZnO, MgO), hydroxides (Mg(OH)₂) or sulfates (CaSO₄) and waxy surfactant. These formulations were subjected to aggressive conditions in order to compare their stability. For that, the viscosity of the formulations was evaluated just after preparation and after being subjected to aggressive conditions (70°C, for 1 h). Table 9 shows that, with the exception of formulations prepared with CaSO₄, all the formulations presented instability due to viscosity increments or phase separation (cellulose sedimentation). These results confirm that neither oxides nor hydroxides of divalent cations are adequate to obtain stable formulations.

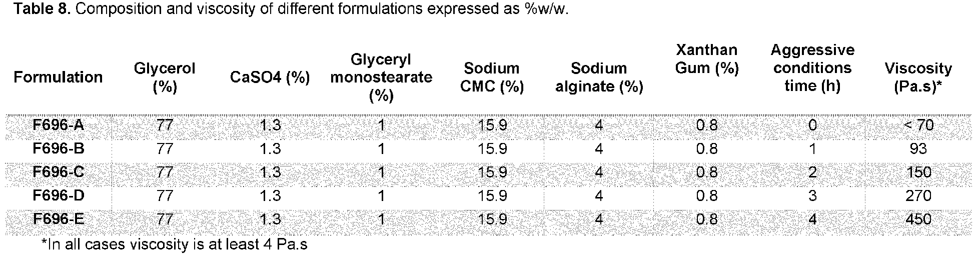

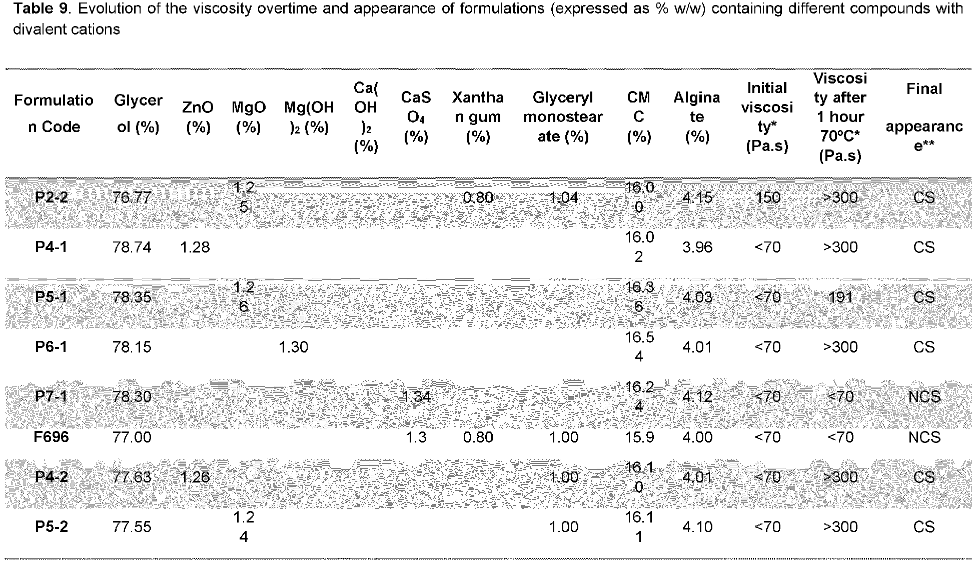

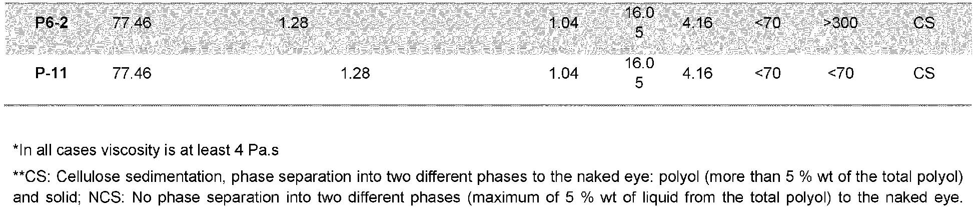

## Claims

1. A composition having a dynamic viscosity from 4 to 300 Pa.s comprising:
(i) a polyol selected from the group consisting of glycerol, propylene glycol and a combination thereof in an amount of at least 60 wt %;
(ii) a swelling polymer selected from the group consisting of celluloses and a derivative thereof or a mixture thereof in an amount of at least 1 wt %, wherein the derivative of celluloses is selected from the group consisting of a salt of carboxymethyl cellulose (CMC), ethylcellulose, hydroxyethyl cellulose, hydroxyethylmethyl cellulose, hydroxypropyl methylcellulose (HPMC) or hypromellose hydroxypropyl cellulose (HPC), low-substituted hydroxypropyl cellulose (L-HPC), methylcellulose, and a combination thereof;
(iii) an inorganic salt comprising divalent cations selected from the group consisting of CaSO₄, CaCl₂, MgCl₂, CuSO₄, and CaCO₃ or a mixture thereof in an amount ranging from 0.01 to 5 wt %; and
(iv) a solid waxy surfactant selected from the group consisting of glyceryl monostearate, sorbitan monostearate, cetostearyl alcohol and combinations thereof
wherein said composition does not comprise more than 3.5 wt % of water, wherein wt % is the weight of each component relative to the total weight of the composition and wherein the ratio component (ii)/component (iii) is from 4 to 250; and wherein the dynamic viscosity is measured at a temperature of 19 ± 2 °C and at 12 rpm as rotational speed.

2. The composition according to claim 1, wherein component (i) is glycerol.

3. The composition according to any one of claims 1 or 2, wherein component (ii) is selected from the group consisting of a salt of carboxymethyl cellulose (CMC), hydroxypropyl methylcellulose (HPMC), hydroxypropyl cellulose (HPC) and a combination thereof.

4. The composition according to any one of claims 1 to 3, further comprising a compound selected from the group consisting of a thickener, a polysaccharide different from component (ii), a surfactant different from component (iv), an adhesive polymer, a fat, and a combination thereof.

5. The composition according to any one of claims 1 to 4 comprising:
(i) 60 to 90 wt % of glycerol
(ii) 5 to 25 wt % of sodium carboxymethylcellulose,
(iii) 0.1 to 2.5 wt % of calcium sulphate
(iv) 0.1 to 7 wt % of glyceryl monostearate
(v) 0 to 2 wt % of xanthan gum
(vi) 0 to 3 wt % of copovidone
(vii) 0 to 10 wt % of sodium alginate; and
(viii) 0 to 10 wt % of *Vitis vinifera* seed oil.

6. The composition according to any one of claims 1 to 5, further comprising at least an active ingredient.

7. The composition according to claim 6, wherein the active ingredient is suitable for topical or oral use and is selected from the group consisting of antibiotics, antimycotics, enzymes, antiprotozoals, antivirals, anti-inflammatories, antihistamines, antiperiodontosis agents, antifungals, antibacterials, whitening agents, disinfectants, analgesics, anesthetics, wound healing agents, anti-nauseants, chemotherapeutics, immunosuppressive agents, immunomodulators, antiseptics, mucolytics, antitussives, decongestants, calcium absorption and regulating agents, hormones, vaccines, biological agents, personal care products, natural plant extracts, vitamins, omega 3- oils, amino acids, peptides and combinations thereof.

8. The composition according to claim 7, wherein the active ingredient is selected from the group consisting of miconazole, carbenoxolone, dexamethasone, triamcinolone, lidocaine, clobetasol, dipotassium glycyrrhizate, dexpanthenol, allantoin, chlorhexidine digluconate and mixtures thereof.

9. The composition according to any one of claims 6 to 8 for use as a medicament.

10. The composition according to any one of claims 6 to 8 for use in the prevention and/or treatment of a disease selected from the group consisting of:
(a) a buccal disease selected from the group consisting of xerostomia, keratosis, desquamative stomatitis (or gingivitis), candidiasis, pericoronitis, erythroplasia, burning mouth syndrome, cancer sores, cold sores, geographic tongue, leukoplakia, lichen planus, erythema multiforme, drug eruptions, halitosis, periodontitis, pemphigus vulgaris, Sjögren's syndrome, gingivitis, ulceration, recurrent aphtous stomatitis and malignant ulcers;
(b) a gastric/gastroesophageal disease selected from the group consisting of gastroesophageal reflux and esophagitis;
(c) an anal disease that is the haemorrhoidal disease; and
(d) a vaginal disease selected from the group consisting of bacterial vaginosis, vaginal yeast infection, vaginitis, chlamydia, gonorrhea, genital herpes, and genital warts.

11. Use of the composition according to any one of claims 1 to 7 as an adhesive for dentures.

12. A method for the preparation of a composition according to any one of claims 1 to 8 comprising:
(a) dispersing component (iii) and component (iv) within component (i); and
(b) dispersing component (ii) in the mixture resulting from the previous step.

13. The method according to claim 12, further comprising at least two additional steps between steps (a) and (b), said steps being:
I. heating the mixture resulting from step (a) at least at the melting temperature of component (iv) and not exceeding a temperature of 70°C; and
II. emulsifying the mixture resulting from step (I) while cooling at least to 40°C.

14. A method for obtaining a nanoparticle composition comprising contacting a composition according to any one of claims 1 to 8 that contains an adhesive polymer dissolved in compound (i), with an aqueous medium, wherein said adhesive polymer is selected from the group consisting of half esters of poly(methyl vinyl ether-co-maleic anhydride) (PVM/MA) copolymers, poly(methylvinyl ether/maleic acid), zein and polymethacrylates.

## Patentansprüche

1. Eine Zusammensetzung mit einer dynamischen Viskosität von 4 bis 300 Pa.s, umfassend:
(i) ein Polyol, ausgewählt aus der Gruppe bestehend aus Glycerin, Propylenglykol und einer Kombination davon in einer Menge von mindestens 60 Gew.-%;
(ii) ein quellbares Polymer, ausgewählt aus der Gruppe bestehend aus Cellulosen und einem Derivat davon oder einer Mischung davon in einer Menge von mindestens 1 Gew.-%, wobei das Derivat von Cellulosen ausgewählt ist aus der Gruppe bestehend aus einem Salz von Carboxymethylcellulose (CMC), Ethylcellulose, Hydroxyethylcellulose, Hydroxyethylmethylcellulose, Hydroxypropylmethylcellulose (HPMC) oder Hypromellose, Hydroxypropylcellulose (HPC), niedrig substituierter Hydroxypropylcellulose (L-HPC), Methylcellulose und einer Kombination davon;
(iii) ein anorganisches Salz umfassend zweiwertige Kationen ausgewählt aus der Gruppe bestehend aus CaSO₄, CaCl₂, MgCl₂, CuSO₄ und CaCO₃ oder einer Mischung davon in einer Menge von 0,01 bis 5 Gew.-%; und
(iv) ein festes wachsartiges Tensid, ausgewählt aus der Gruppe bestehend aus Glycerylmonostearat, Sorbitanmonostearat, Cetostearylalkohol und Kombinationen davon
wobei die Zusammensetzung nicht mehr als 3,5 Gew.-% Wasser umfasst, wobei Gew.-% das Gewicht jeder Komponente relativ zum Gesamtgewicht der Zusammensetzung ist und wobei das Verhältnis der Komponente (ii) zur Komponente (iii) zwischen 4 und 250 liegt; und wobei die dynamische Viskosität bei einer Temperatur von 19 ± 2 °C und einer Drehzahl von 12 U/min gemessen wird.

2. Die Zusammensetzung nach Anspruch 1, wobei die Komponente (i) Glycerol ist.

3. Die Zusammensetzung nach einem der Ansprüche 1 oder 2, wobei die Komponente (ii) ausgewählt ist aus der Gruppe bestehend aus einem Salz der Carboxymethylcellulose (CMC), Hydroxypropylmethylcellulose (HPMC), Hydroxypropylcellulose (HPC) und einer Kombination davon.

4. Die Zusammensetzung nach einem der Ansprüche 1 bis 3, weiterhin umfassend eine Verbindung ausgewählt der Gruppe bestehend aus einem Verdicker, einem Polysaccharid anders als Komponente (ii), einem Tensid anders als Komponente (iv), einem adhäsiven Polymer, einem Fett und einer Kombination davon.

5. Die Zusammensetzung nach einem der Ansprüche 1 bis 4 umfassend:
(i) 60 bis 90 Gew.-% von Glycerol
(ii) 5 bis 25 Gew.-% Natriumcarboxymethylcellulose,
(iii) 0,1 bis 2,5 Gew.-% Calciumsulfat
(iv) 0,1 bis 7 Gew.-% des Glycerylmonostearats
(v) 0 bis 2 Gew.-% Xanthangummi
(vi) 0 bis 3 Gew.-% des Copovidon
(vii) 0 bis 10 Gew.-% Natriumalginat; und
(viii) 0 bis 10 Gew.-% *Vitis vinifera* Samenöl.

6. Die Zusammensetzung nach einem der Ansprüche 1 bis 5, weiterhin umfassend mindestens einen Wirkstoff.

7. Die Zusammensetzung nach Anspruch 6, wobei der Wirkstoff für die topische oder orale Anwendung geeignet ist und aus der Gruppe ausgewählt ist bestehend aus Antibiotika, Antimykotika, Enzymen, Antiprotozoika, Virostatika, Entzündungshemmern, Antihistaminika, Antiparodontose-Mitteln, Antipilzmittel, Antibakterielle Mittel, Bleichmitteln, Desinfektionsmitteln, Analgetika, Anästhetika, Wundheilungsmitteln, Antiemetika, Chemotherapeutika, Immunsuppressiva, Immunmodulatoren, Antiseptika, Mukolytika, Antitussiva, Dekongestiva, Calciumabsorptions- und -regulationsmitteln, Hormonen, Impfstoffen, biologischen Wirkstoffen, Körperpflegeprodukten, natürlichen Pflanzenextrakten, Vitaminen, Omega-3-Ölen, Aminosäuren, Peptiden und Kombinationen davon.

8. Die Zusammensetzung nach Anspruch 7, wobei der Wirkstoff ausgewählt ist aus der Gruppe bestehend aus Miconazol, Carbenoxolon, Dexamethason, Triamcinolon, Lidocain, Clobetasol, Dikaliumglycyrrhizat, Dexpanthenol, Allantoin, Chlorhexidin-Diglukonat und Mischungen davon.

9. Die Zusammensetzung nach einem der Ansprüche 6 bis 8 zur Verwendung als Medikament.

10. Die Zusammensetzung nach einer der Ansprüche 6 bis 8 zur Verwendung in der Prävention und/oder Behandlung einer Krankheit ausgewählt aus der Gruppe bestehend aus:
(a) eine bukkale Erkrankung, ausgewählt aus der Gruppe bestehend aus Xerostomie, Keratose, desquamative Stomatitis (oder Gingivitis), Candidiasis, Perikoronitis, Erythroplasie, Burning-Mouth-Syndrom, Krebsgeschwüren, Lippenbläschen, Landkartenzunge, Leukoplakie, Lichen planus, Erythema multiforme, Arzneimittelexanthemen, Halitosis, Parodontitis, Pemphigus vulgaris, Sjögren-Syndrom, Gingivitis, Ulzerationen, rezidivierender aphthöser Stomatitis und bösartige Geschwüre;
(b) eine Magen-/gastroösophageale Erkrankung, ausgewählt aus der Gruppe bestehend aus gastroösophagealem Reflux und Ösophagitis;
(c) eine Analkrankheit, nämlich Hämorrhoiden; und
(d) eine Vaginalerkrankung, ausgewählt aus der Gruppe bestehend aus bakterieller Vaginose, vaginaler Hefepilzinfektion, Vaginitis, Chlamydien, Gonorrhö, Genitalherpes und Genitalwarzen.

11. Eine Verwendung der Zusammensetzung nach einem der Ansprüche 1 bis 7 als Haftmittel für Zahnersatz.

12. Ein Verfahren zur Herstellung einer Zusammensetzung nach einem der Ansprüche 1 bis 8, umfassend:
(a) Dispergieren der Komponente (iii) und der Komponente (iv) in der Komponente (i); und
(b) Dispergieren der Komponente (ii) in der aus dem vorherigen Schritt resultierenden Mischung.

13. Das Verfahren nach Anspruch 12, das zusätzlich mindestens zwei weitere Schritte zwischen den Schritten (a) und (b) umfasst, wobei diese Schritte wie folgt lauten:
I. Erhitzen der aus Schritt (a) resultierenden Mischung auf mindestens die Schmelztemperatur der Komponente (iv) und unter Einhaltung einer Temperatur von maximal 70 °C; und
II. Emulgieren der aus Schritt (I) resultierenden Mischung unter Abkühlen auf mindestens 40 °C.

14. Ein Verfahren zur Herstellung einer Nanopartikelzusammensetzung, umfassend das Inkontaktbringen einer Zusammensetzung nach einem der Ansprüche 1 bis 8, die ein in Komponente (i) gelöstes adhäsives Polymer enthält, mit einem wässrigen Medium, wobei das adhäsive Polymer ausgewählt ist aus der Gruppe bestehend aus Halb-Estern von Poly(methylvinylether-alt-maleinsäureanhydrid) (PVM/MA)-Copolymeren, Poly(methylvinylether/maleinsäure), Zein und Polymethacrylaten.

## Revendications

1. Composition ayant une viscosité dynamique comprise entre 4 et 300 Pa.s, comprenant :
(i) un polyol choisi dans le groupe constitué par le glycérol, le propylène glycol et une combinaison de ceux-ci en une quantité d'au moins 60 % en poids ;
(ii) un polymère gonflant choisi dans le groupe constitué par les celluloses et un dérivé de celles-ci ou un mélange de celles-ci en une quantité d'au moins 1 % en poids, dans laquelle le dérivé des celluloses est choisi dans le groupe constitué par un sel de carboxyméthyl cellulose (CMC), l'éthylcellulose, l'hydroxyéthyl cellulose, l'hydroxyéthylméthylcellulose, l'hydroxypropylméthylcellulose (HPMC) ou l'hypromellose, l'hydroxypropylcellulose (HPC), l'hydroxypropylcellulose faiblement substituée (L-HPC), la méthylcellulose, et une combinaison de ceux-ci ;
(iii) un sel inorganique comprenant des cations divalents choisis dans le groupe constitué de CaSO₄, CaCl₂, MgCl₂, CuSO₄ et CaCO₃ ou un mélange de ceux-ci en une quantité comprise entre 0,01 et 5 % en poids ; et
(iv) un tensioactif cireux solide choisi dans le groupe constitué par le monostéarate de glycéryle, le monostéarate de sorbitane, l'alcool cétostéarylique et leurs combinaisons
dans laquelle ladite composition ne comprend pas plus de 3,5 % en poids d'eau, dans laquelle le pourcentage en poids est le poids de chaque composant par rapport au poids total de la composition et dans laquelle le rapport composant (ii)/composant (iii) est compris entre 4 et 250 ; et dans laquelle la viscosité dynamique est mesurée à une température de 19 + 2 °C et à une vitesse de rotation de 12 tr/min.

2. La composition selon la revendication 1, dans laquelle le composant (i) est le glycérol.

3. La composition selon l'une quelconque des revendications 1 ou 2, dans laquelle le composant (ii) est choisi parmi le groupe constitué d'un sel de carboxyméthylcellulose (CMC), d'hydroxypropylméthylcellulose (HPMC), d'hydroxypropylcellulose (HPC) et d'une combinaison de ceux-ci.

4. La composition selon l'une quelconque des revendications 1 à 3, comprenant en outre un composé choisi dans le groupe constitué d'un épaississant, d'un polysaccharide différent du composant (ii), d'un tensioactif différent du composant (iv), d'un polymère adhésif, d'une graisse et d'une combinaison de ceux-ci.

5. La composition selon l'une quelconque des revendications 1 à 4 comprenant :
(i) 60 à 90 % en poids de glycérol
(ii) 5 à 25 % en poids de carboxyméthylcellulose sodique,
(iii) 0,1 à 2,5 % en poids de sulfate de calcium
(iv) 0,1 à 7 % en poids de monostéarate de glycéryle
(v) 0 à 2 % en poids de gomme xanthane
(vi) 0 à 3 % en poids de copovidone
(vii) 0 à 10 % en poids d'alginate de sodium ; et
(viii) 0 à 10 % en poids d'huile de pépins de *Vitis vinifera.*

6. Composition selon l'une quelconque des revendications 1 à 5, comprenant en outre au moins un ingrédient actif.

7. Composition selon la revendication 6, dans laquelle l'ingrédient actif est adapté pour une utilisation topique ou orale et est choisi dans le groupe constitué par les antibiotiques, les antimycotiques, les enzymes, les antiprotozoaires, les antiviraux, les anti-inflammatoires, les antihistaminiques, les agents anti-parodontose, les antifongiques, les antibactériens, les agents blanchissants, les désinfectants, les analgésiques, les anesthésiques, les agents cicatrisants, les anti-nauséeux, les chimiothérapeutiques, les agents immunosuppresseurs, les immunomodulateurs, les antiseptiques, les mucolytiques, les antitussifs, les décongestionnants, les agents d'absorption et de régulation du calcium, les hormones, les vaccins, les agents biologiques, les produits de soins personnels, les extraits de plantes naturelles, les vitamines, les huiles oméga 3, les acides aminés, les peptides et leurs combinaisons.

8. Composition selon la revendication 7, dans laquelle l'ingrédient actif est choisi dans le groupe constitué par le miconazole, la carbénoxolone, la dexaméthasone, la triamcinolone, la lidocaïne, le clobétasol, le glycyrrhizate dipotassique, le dexpanthénol, l'allantoïne, le digluconate de chlorhexidine et leurs mélanges.

9. Composition selon l'une quelconque des revendications 6 à 8, pour utilisation comme médicament.

10. Composition selon l'une quelconque des revendications 6 à 8, pour utilisation dans la prévention et/ou le traitement d'une maladie choisie dans le groupe constitué par :
(a) une maladie buccale choisie dans le groupe constitué par la xérostomie, la kératose, la stomatite desquamative (ou gingivite), la candidose, la péricoronarite, l'érythroplasie, le syndrome de la bouche brûlante, les aphtes, l'herpès labial, la langue géographique, la leucoplasie, le lichen plan, l'érythème polymorphe, les éruptions médicamenteuses, l'halitose, la parodontite, le pemphigus vulgaire, le syndrome de Sjögren, la gingivite, l'ulcération, la stomatite aphteuse récidivante et les ulcères malins ;
(b) une maladie gastrique/gastro-œsophagienne choisie dans le groupe constitué par le reflux gastro-œsophagien et l'œsophagite ;
(c) une maladie anale qui est la maladie hémorroïdaire ; et
(d) une maladie vaginale choisie dans le groupe constitué par la vaginose bactérienne, l'infection vaginale à levures, la vaginite, la chlamydia, la gonorrhée, l'herpès génital et les verrues génitales.

11. Utilisation de la composition selon l'une quelconque des revendications 1 à 7 comme adhésif pour prothèses dentaires.

12. Procédé de préparation d'une composition selon l'une quelconque des revendications 1 à 8, comprenant :
(a) la dispersion du composant (iii) et du composant (iv) dans le composant (i) ; et
(b) la dispersion du composant (ii) dans le mélange résultant de l'étape précédente.

13. Procédé selon la revendication 12, comprenant en outre au moins deux étapes supplémentaires entre les étapes (a) et (b), lesdites étapes étant :
I. le chauffage du mélange résultant de l'étape (a) au moins à la température de fusion du composant (iv) et sans dépasser une température de 70 °C; et
II. l'émulsification du mélange résultant de l'étape (I) tout en refroidissant à au moins 40 °C.

14. Procédé pour obtenir une composition de nanoparticules comprenant la mise en contact d'une composition selon l'une quelconque des revendications 1 à 8 qui contient un polymère adhésif dissous dans le composé (i), avec un milieu aqueux, dans lequel ledit polymère adhésif est choisi dans le groupe constitué par les demi-esters de copolymères de poly(méthylvinyléther-co-anhydride maléique) (PVM/MA), le poly(méthylvinyléther/acide maléique), la zéine et les polyméthacrylates.
